(19) Europäisches Patentamt
European Patent Office
Office européen des brevets

(11) EP 3 209 267 B1

(12) # EUROPÄISCHE PATENTSCHRIFT

(45) Veröffentlichungstag und Bekanntmachung des Hinweises auf die Patenterteilung:
**28.11.2018 Patentblatt 2018/48**

(21) Anmeldenummer: **15767477.1**

(22) Anmeldetag: **22.09.2015**

(51) Int Cl.:
***A61K 8/26*** (2006.01) ***A61K 8/28*** (2006.01)
***A61K 8/37*** (2006.01) ***A61Q 15/00*** (2006.01)
***A61K 8/893*** (2006.01) ***A61K 8/02*** (2006.01)

(86) Internationale Anmeldenummer:
**PCT/EP2015/071778**

(87) Internationale Veröffentlichungsnummer:
**WO 2016/062487 (28.04.2016 Gazette 2016/17)**

(54) **LEISTUNGSGESTEIGERTE ANTITRANSPIRANTIEN**

IMPROVED ANTITRANSPIRANTS

ANTIPÉRSPIRANTES AMELIORÉES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.10.2014 DE 102014221531**

(43) Veröffentlichungstag der Anmeldung:
**30.08.2017 Patentblatt 2017/35**

(73) Patentinhaber: **Henkel AG & Co. KGaA**
**40589 Düsseldorf (DE)**

(72) Erfinder:
- **DÖRING, Thomas**
**41540 Dormagen (DE)**
- **HODES, Jing (Leo)**
**58093 Hagen (DE)**

(56) Entgegenhaltungen:
**WO-A2-2010/046291**
**WO-A2-2014/095689**
**DE-A1-102010 000 746**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 209 267 B1

## Beschreibung

Die vorliegende Anmeldung betrifft kosmetische und dermatologische Antitranspirant-Stifte mit verbesserter schweißhemmender Wirksamkeit und verbesserter Textur.

Zusammensetzungen gegen Körpergeruch sind ein wichtiger Bestandteil der täglichen persönlichen Hygiene. Sie sollen dafür sorgen, dass der im Laufe des Tages durch diverse Aktivitäten (körperliche Bewegung, Arbeit, Sport), aber auch durch psychische Belastung gebildete Schweiß nicht zu unangenehmem Körpergeruch führt. Genau so vielfältig, wie die Bestandteile des Schweißes und die Ursachen für die Körpergeruchsentwicklung sind, sind auch die deodorierenden Wirkstoffe der handelsüblichen Deodorantien. Als kosmetische deodorierende Wirkstoffe einsetzbar sind Geruchsabsorber, Duftstoffe, desodorierend wirkende Ionenaustauscher, keimhemmende Mittel, präbiotisch wirksame Komponenten sowie Enzyminhibitoren. Körpergeruch ist, vereinfacht dargestellt, auf die bakterielle Zersetzung der organischen Bestandteile des Schweißes zurückzuführen. Für die bakterielle Zersetzung wiederum sind einige der für die natürliche Mikroflora der menschlichen Haut typischen Bakterien verantwortlich, insbesondere Gram-positive anaereobe Kokken, z. B. Staphylococcen, wie Staphylococcus hominis, und Corynebakterien. Da der Körpergeruch durch bakterielle Tätigkeit entsteht, kann er besonders wirksam durch die Anwendung kosmetischer Mittel (Seifen, Cremes, Puder, Stifte, Roller, Gele oder Sprays) verhindert werden, die antimikrobiell wirksame Stoffe und Parfümölkompositionen enthalten.

Als Applikationsformen für besagte Zusammensetzungen haben sich das Aerosolspray, der Roll-on sowie Antitranspirant-Stifte am Markt etabliert. Ferner sind auch das Deodorant in Puderform (auch als kompaktiertes Puder) oder das auf einem wegwerfbaren Substrat (wie einem Tuch, Pad oder Bausch) aufgebrachte Deodorant, bekannt. Als besonders angenehme Anwendungsform kennt der Fachmann die sogenannten Antitranspirant-Stifte oder Creme-Stifte ("soft solids"). Darunter werden viskose Zusammensetzungen verstanden, die eine cremige Textur besitzen und vor der Anwendung durch ein oder mehrere Öffnungen einer Abgabevorrichtung eines Applikators herausgedrückt werden. Antitranspirant-Stifte werden in der Regel wasserarm oder wasserfrei konfektioniert. Die wasserfreie Konfektionierung wird insbesondere dann gegenüber wässrigen Systemen bevorzugt, wenn eine besonders hohe schweißhemmende Wirksamkeit erwünscht ist, da besonders effektive schweißhemmende Wirkstoffe wie beispielsweise aktiviertes Aluminiumchlorhydrat in wässrigen Medien nicht langzeitstabil sind.

Bei wasserarm, bevorzugt wasserfrei, konfektionierten Antitranspirantien stellt die optimale Freisetzung der schweißhemmenden Wirkstoffe am Wirkort eine ganz besondere Herausforderung dar. Die in Antitranspirantien dieses Typs üblicherweise eingesetzte Wachsmatrix umgibt die antitranspirant wirkenden Aluminium- und/oder Aluminium/Zirkonium-Salze. Hierdurch wird deren Kontakt mit der Wasserphase eingeschränkt oder sogar verhindert. Bei Applikation des Antitranspirants auf der Haut können sich die Wirkstoffe dann nicht in ausreichender Menge in der Wasserphase (Schweiß) lösen, wodurch die schweißhemmende Wirksamkeit der Mittel stark beeinträchtigt wird.

Der Fachmann versucht daher stets die Freisetzung der schweißhemmenden Wirkstoffe zu steigern und/oder den Wirkungsgrad der schweißhemmenden Wirkstoffe zu erhöhen. Zur Verbesserung der Freisetzung wurden bisher amphiphile (grenzflächenaktive) Stoffe eingesetzt. Durch den Einsatz dieser amphiphilen Stoffe kann die Hydrophobizität der Wachsmatrix herabgesetzt und damit die Wirkstoffverfügbarkeit gesteigert werden. Die auf diese Weise erzielten Verbesserungen wirken jedoch nur kurzfristig, da amphiphile Stoffe gleichzeitig auch den Eintrag von Wasser in die wasserfreie Zusammensetzung fördern. Hierdurch wird wiederum mit der Zeit eine Verhärtung der Oberfläche des stiftförmigen Antitranspant-Mittels hervorgerufen. Aus dieser Verhärtung resultiert eine mangelnde Produktabgabe am Wirkort, was zu einer Verschlechterung der schweißhemmenden Wirkung führt. Darüber hinaus beeinträchtigt eine durch Wassereintrag verkrustete Oberfläche sehr stark auch weitere kosmetischen Produkteigenschaften. Das Antitranspirant-Mittel verliert seine cremige Textur, und die an der Oberfläche des Mittels befindlichen Verhärtungen können Missempfindungen auf Seiten des Anwenders hervorrufen.

Zur Steigerung der Wirksamkeit von Antitranspirant-Produkten werden oft die Gehalte an Aluminiumsalzen deutlich erhöht. Diese Praxis ist nicht nur unwirtschaftlich, sondern kann darüber hinaus zu Hautunverträglichkeiten führen.

Es war daher die Aufgabe der vorliegenden Erfindung, eine wasserarme, bevorzugt wasserfreie, kosmetische Zusammensetzung gegen Körpergeruch bereitzustellen, die eine verbesserte Wirksamkeit der schweißhemmenden Wirkstoffe besitzt. Die Freisetzung der schweißhemmenden Wirkstoffe am Wirkort sollte ebenso gesteigert werden. Gleichzeitig sollten auch die Anwendungseigenschaften des Mittels hinsichtlich seiner Textur-wie beispielsweise seine cremige Konsistenz und die Gleitfähigkeit des Mittels nach wiederholter Anwendung auf der Haut - verbessert und auf diese Weise eine gleichmäßige Produktabgabe gewährleistet werden.

In überraschender Weise hat sich nun gezeigt, dass sich diese Aufgabe in hervorragender Weise lösen lässt, wenn in Antitranspirant-Stiften ein schweißhemmender Wirkstoff in Kombination mit mindestens einem alkoxy-modifizierten Silikon und mindestens einem Ester aus einer C8-C14-Alkansäure und einem C8-C14-Alkanol eingesetzt wird.

Gegenstand der vorliegenden Erfindung ist in einer ersten Ausführungsform ein Antitranspirant-Stift, enthaltend

(A) mindestens ein alkoxy-modifiziertes Silikon,

(B) mindestens einen Ester aus einer $C_8$-$C_{14}$-Alkansäure und einem $C_8$-$C_{14}$-Alkanol und

(C) mindestens einen schweißhemmenden Wirkstoff aus der Gruppe der Aluminium- oder Aluminium-Zirkonium-Salze.

Als Antitranspirant-Stifte werden im Rahmen dieser Erfindung hochviskose, wasserarm bzw. wasserfrei konfektionierte Zusammensetzungen verstanden. Bei dieser Zusammensetzung kann es sich um eine Dispersion von feinteiligen adstringierenden Aluminium- oder Zirkoniumsalzen in einer durch Gelbildner oder wachsartige Fettkomponenten verfestigten unpolaren Trägerflüssigkeit handeln. Als unpolare Trägerflüssigkeiten können z.B. Kohlenwasserstoffe oder Silikonöle, bevorzugt solche mit Siedepunkten unterhalb von 200°C oder mit einer gewissen Flüchtigkeit, z.B. cyclische Siloxane (Cyclomethicone) und deren Gemische mit hautweichmachenden Ölkomponenten eingesetzt werden. Die Zusammensetzung werden üblicherweise in Stift-, Zylinder oder Kegelform gegossen bzw. gepresst und in einer entsprechenden Umhüllung verpackt angeboten, aus der sie kurz vor der Anwendung herausgedreht oder gedrückt werden. Unter den erfindungsgemäßen Antitranspirant-Stiften, welche die wesentlichen Inhaltsstoffe (A), (B) und (C) enthalten, werden die - bevorzugt in Stift-, Zylinder oder Kegelform gegossen bzw. geformten - Zusammensetzungen ohne weiteres Verpackungs- oder Umhüllungsmaterial verstanden.

Als ersten erfindungswesentlichen Inhaltsstoff (A) enthalten die Antitranspirant-Stifte der vorliegenden Erfindung mindestens ein alkoxy-modifiziertes Silikon. Silikone sind polymere Verbindungen, die nach dem Schema (R2SiO)x aufgebaut sind, wobei R üblicherweise für einen organischen Rest, oft eine Alkylgruppe oder auch eine substituierte Alkylgruppe, steht. Silikone werden auch als Polyorganosilixane bezeichnet, sie können linear oder verzweigt sein. Verknüpft sind die Siliciumatome über Sauerstoffatome in einer Si-O-Si-Bindung.

Unter alkoxy-modifizierten Silikonen werden Silikone verstanden, welche mindestens eine AlkoxyGruppe tragen. Unter Alkoxygruppen werden $C_2$-$C_{10}$-Alkoxygruppen verstanden. Die Alkoxygruppe kann sich endständig am Silikon befinden (d.h. zum Beispiel als Gruppe -O-$CH_3$ oder als Gruppe -O-$CH_2$-$CH_3$ vorliegen). Ebenso ist es aber erfindungsgemäß, wenn die Alkoxygruppe selbst noch einen Substituenten trägt; in diesem Fall wird unter einer Alkyoxy-Modifizierung eine am Silikon befindliche Gruppierung wie beispielsweise (-CH2-CH2-O-), (-CH2-CH2-CH2-O-), (-CH(CH3)-CH2-O-), (-CH2-CH(CH3)-CH2-O-) oder (-CH2-CH2-CH2-CH2-O-) verstanden. Bevorzugt tragen die erfindungsemäßen alkoxy-modifizierten Silikone (A) mindestens eine Gruppierung (-CH2-CH2-O-) und/oder (-CH2-CH2-CH2-O-).

Die Alkoxygruppen können entweder über ein Kohlenstoffatom oder über ein Sauerstoffatom mit dem Silikon verknüpft sein, beispielsweise können die Silikone die Struktureinheiten der Formel (a), (b), (c) und/oder (d) tragen:

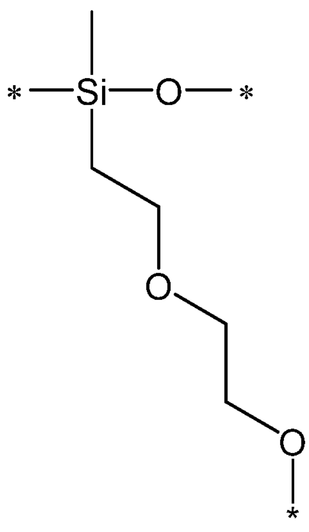
(a)

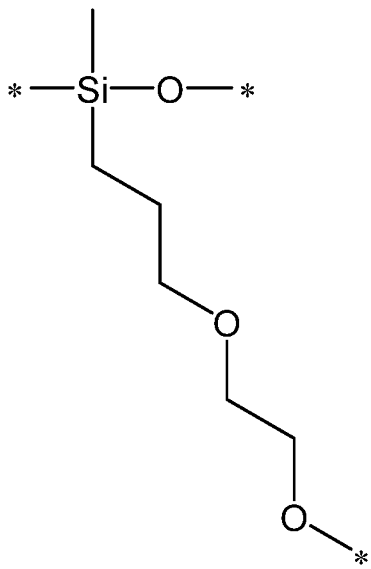
(b)

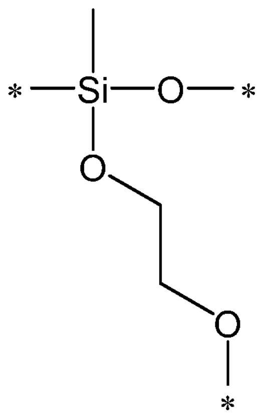

(c)

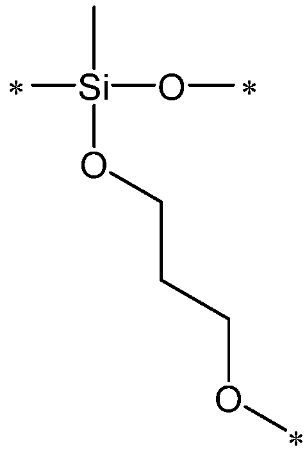

(d)

Besonders bevorzugt ist es, wenn das bzw. die alkoxy-modifizierten Silikone (A) mehr als eine AlkoxyGruppe tragen, d.h. wenn die Silikone (A) polyalkoxyliert sind. Polyalkoxylierte Silikone tragen als Struktureinheiten Polyoxyalkylengruppen, insbesondere Polyoxyethylengruppen (d.h. Gruppen des Typs [-CH2-CH2-O-]$_m$) und/oder Poloxypropylengruppen (d.h. Gruppen des Typs [-CH(CH3)-CH2-O-]$_m$ und/oder [-CH2-CH2-CH2-O-]$_m$). Bevorzugt liegt die Zahl der Polyoxyalkyleineinheiten im SilikonPolymer liegt bei mindestens 2.

Besonders bevorzugt handelt es sich bei dem alkoxy-modifizierten Silikon (A) um ein nichtionisches Silikon. Nichtionische Silikone tragen weder positive noch negative Ladungen.

In einer weiteren Ausführungsform bevorzugt ist ein Antitranspirant-Stift, enthaltend

(A) mindestens ein nichtionisches, polyalkoxyliertes Silikon

(B) mindestens einen Ester aus einer $C_8$-$C_{14}$-Alkansäure und einem $C_8$-$C_{14}$-Alkanol und

(C) mindestens einen schweißhemmenden Wirkstoff aus der Gruppe der Aluminium- oder Aluminium-Zirkonium-Salze.

Als besonders bevorzugt haben sich die alkoxy-modifizierten Silikone (A) erwiesen, welche mindestens eine Struktureinheit der nachfolgend dargestellten Formel (A1) besitzen,

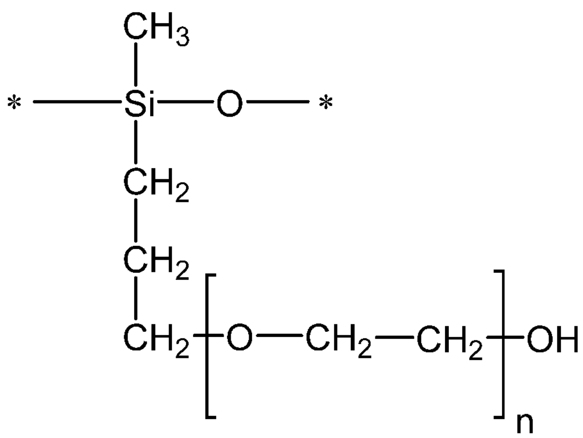

(A1)

worin n für eine ganze Zahl von 2 bis 20, bevorzugt für eine ganze Zahl von 4 bis 18, weiter bevorzugt für eine ganze Zahl von 6 bis 16, noch weiter bevorzugt für eine ganze Zahl von 8 bis 14 und ganz besonders bevorzugt für die Zahl 12 steht.

Wurden Silikone (A) mit mindestens einer Struktureinheit (A1) in den erfindungsgemäßen Antitranspirant-Stiften eingesetzt, so konnte eine besonders effektive Hemmung der Schweißbildung beobachtet wurden. Gleichzeitig zeichneten sich diese Antitranspirant-Stifte durch eine besonders cremige Textur aus.

In einer bevorzugten Ausführungsform ist ein erfindungsgemäßer Antitranspirant-Stift daher dadurch gekennzeichnet, dass er ein alkoxy-modifiziertes Silikon (A) enthält, welches mindestens eine Struktureinheit der Formel (A1) besitzt,

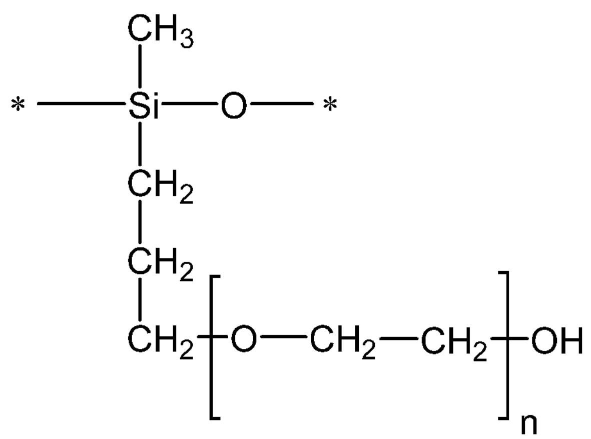

(A1)

worin n für eine ganze Zahl von 2 bis 20, bevorzugt für eine ganze Zahl von 4 bis 18, weiter bevorzugt für eine ganze Zahl von 6 bis 16, noch weiter bevorzugt für eine ganze Zahl von 8 bis 14 und ganz besonders bevorzugt für die Zahl 12 steht.

Ein bevorzugtes erfindungsgemäßes Silikon (A) kann neben einer oder mehrerer Struktureinheiten der allgemeinen Formel (A1) auch weitere Struktureinheiten enthalten, die sich strukturell von den Einheiten der Formel (A1) unterscheiden. Besonders bevorzugt umfasst das Silikon (A) zusätzlich ein oder mehrere Dimethylsiloxaneinheiten. Je nachdem, ob das erfindungsgemäße Silikon linear oder verzweigt ist, besitzt es zwei (im Falle eines kettenförmigen, linearen Silikons) oder mehrere (im Falle eines verzweigten Silikons) Endgruppen. Als besonders vorteilhaft hat es sich herausgestellt, wenn ein erfindungsgemäßes Silikone (A) als Endruppen jeweils eine Trimethylsilyloxy-Gruppe (d.h. eine Gruppe -O-Si$(CH_3)_3$) besitzt.

In einer weiteren ganz besonders bevorzugten Ausführungsform ist ein erfindungsgemäßer Antitranspirant-Stift daher dadurch gekennzeichnet, dass er ein alkoxy-modifiziertes Silikon (A) enthält, welches sich aus Struktureinheiten der Formel (A1), der Formel (A2), der Formel (A3) und der Formel (A3') zusammensetzt,

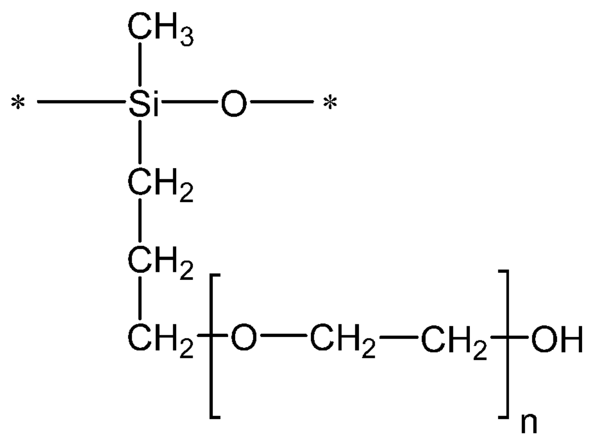

(A1)

worin n - unabhängig in jeder Struktureinheit (A1) - jeweils für eine ganze Zahl von 2 bis 20, bevorzugt für eine ganze Zahl von 4 bis 18, weiter bevorzugt für eine ganze Zahl von 6 bis 16, noch weiter bevorzugt für eine ganze Zahl von 8 bis 14 und ganz besonders bevorzugt für die Zahl 12 steht,

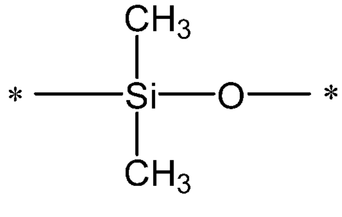

(A2)

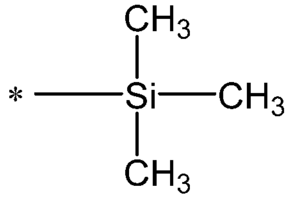

(A3).

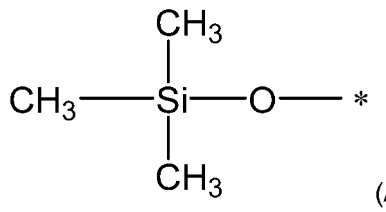

(A3').

Unter einem Silikon (A), das sich aus Struktureinheiten der Formel (A1), der Formel (A2), der Formel (A3) und der Formel (A3') zusammensetzt, wird in diesem Zusammenhang ein Silikon verstanden, welches ausschließlich (jeweils eine oder mehrere) Struktureinheiten der Formeln (A1), (A2), (A3) und (A3') besitzt. Hierbei kann das Silikon auch verschiedene Struktureinheiten der Formel (A1) enthalten, die sich jeweils durch ihre Zahl n unterscheiden.

Die in den vorgenannten Struktureinheiten mit einem Stern gekennzeichneten Positionen stellen jeweils die Verknüpfungsstellen zu den anderen Struktureinheiten dar. Beispielsweise kann ein ganz besonders bevorzugtes Silikon, welches sich aus Struktureinheiten der Formel (A1), der Formel (A2), der Formel (A3) und der Formel (A3') zusammensetzt, die folgende Struktur besitzen:

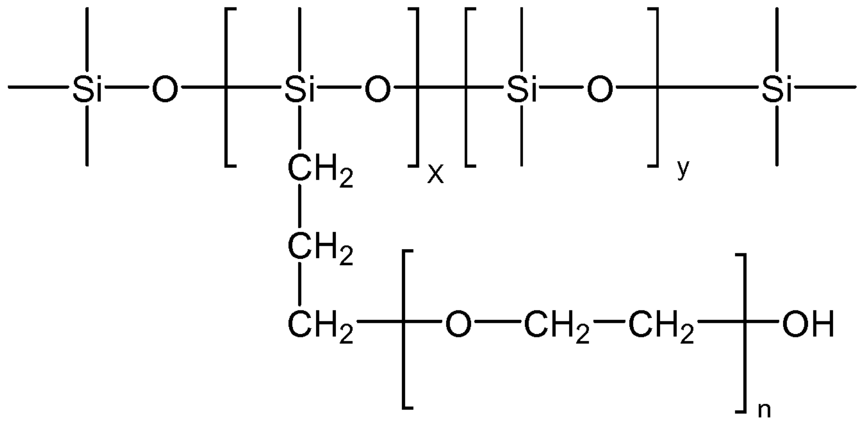

x und y werden hierbei abhängig vom gewünschten Molekulargewicht des Silikons gewählt, und n stellt eine der oben beschriebenen erfindungsgemäßen, bevorzugten oder besonders bevorzugten ganzen Zahlen dar.

Als Silikone (A) können sowohl niedermolekulare als auch höhermolekulare alkoxy-modifizierte Silikone eingesetzt werden. Besonders vorteilhafte Effekte wurden bei Silikonen (A) mit einer Molmasse von 800 bis 10 000 g/mol, bevorzugt von 1 000 bis 9 000 g/mol, weiter bevorzugt von 2 000 bis 8 000 g/mol und besonders bevorzugt von 2 500 bis 5 000 g/mol beobachtet.

Wenn in den erfindungsgemäßen Antitranspirant-Stiften Silikone (A) mit einer Molmasse von 2 500 bis 5 000 g/mol eingesetzt wurden, zeichneten sich die Stifte auch nach wiederholter Anwendung durch eine besonders cremige Textur aus.

In einer weiteren ganz besonders bevorzugten Ausführungsform ist ein erfindungsgemäßer Antitranspirant-Stift daher dadurch gekennzeichnet, dass er mindestens ein alkoxy-modifiziertes Silikon (A) mit einer Molmasse von 800 bis 10 000 g/mol, bevorzugt von 1 000 bis 9 000 g/mol, weiter bevorzugt von 2 000 bis 8 000 g/mol und besonders bevorzugt von 2 500 bis 5 000 g/mol enthält. Besonders gut geeignete Silikone sind beispielsweise:

Abil B 8843 der Firma Evonic, PEG-14 DIMETHICONE
Xiameter OFX 0193 Fluid der Firma Dow Corning, PEG-12 Dimethicone

Als zweiten wesentlichen Inhaltsstoff (B) enthalten die erfindungsgemäßen Antitranspirant-Stifte mindestens einen Ester aus einer $C_8$-$C_{14}$-Alkansäure und einem $C_8$-$C_{14}$-Alkanol.

Bei den $C_8$-$C_{14}$-Alkansäuren handelt es sich um lineare Alkansäuren. Beispiele für bevorzugte $C_8$-$C_{14}$-Alkansäuren sind Caprylsäure (1-Octansäure) $C_7H_{15}COOH$, Pelargonsäure (1-Nonansäure) $C_8C_{17}COOH$, Caprinsäure (1-Decansäure) $C_9C_{19}COOH$, 1-Undedansäure $C_{10}H_{21}COOH$, Laurinsäure (1-Dodecansäure) $C_{11}H_{23}COOH$, 1-Tridecansäure $C_{12}H_{25}COOH$ und Myristinsäure (1-Tetradecansäure) $C_{13}H_{27}COOH$.

Besonders bevorzugt ist Myristinsäure.

Bei den $C_8$-$C_{14}$-Alkanolen handelt es sich um lineare Alkanole. Erfindungsgemäße $C_8$-$C_{14}$-Akanole sind 1-Otanol, 1-Nonanol, 1-Decanol, 1-Undedanol, 1-Dodecanol (Laurylalkohol), 1-Tridecanol und 1-Tetradecanol (Myristylalkohol).

Besonders bevorzugt ist Myristylalkohol.

Bei den Estern aus einer $C_8$-$C_{14}$-Alkansäure und einem $C_8$-$C_{14}$-Alkanol handelt es sich bevorzugt um Verbindungen der folgenden Struktur (B1):

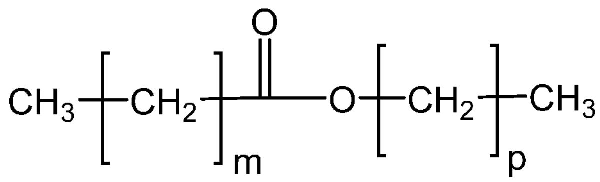

(B1)

worin

m für eine ganze Zahl von 6 bis 12, bevorzugt für eine ganze Zahl von 8 bis 12 und besonders bevorzugt für eine ganze Zahl von 10 bis 12 steht und
p für eine ganze Zahl von 7 bis 13, bevorzugt für eine ganze Zahl von 9 bis 13 und besonders bevorzugt für eine ganze Zahl von 11 bis 13 steht.

In einer weiteren ganz besonders bevorzugten Ausführungsform ist ein erfindungsgemäßer Antitranspirant-Stift daher dadurch gekennzeichnet, dass er einen Ester (B) der Formel (B1) enthält,

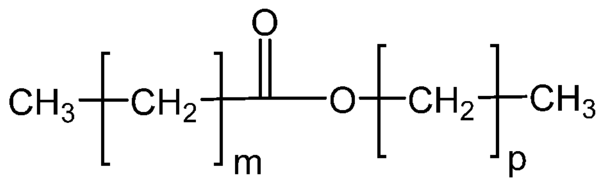

(B1)

worin

m für eine ganze Zahl von 6 bis 12, bevorzugt für eine ganze Zahl von 8 bis 12 und besonders bevorzugt für eine ganze Zahl von 10 bis 12 steht und
p für eine ganze Zahl von 7 bis 13, bevorzugt für eine ganze Zahl von 9 bis 13 und besonders bevorzugt für eine ganze Zahl von 11 bis 13 steht.

Überaschenderweise hat es sich als besonders vorteilhaft herausgestellt, wenn die Ester (B) durch Veresterung einer Alkansäure mit einem Alkohol der gleichen Kettenlänge hergestellt werden. Mit anderen Worten wurden besonders gute Ergebnisse im Hinblick auf Cremigkeit und Freisetzung des Antitranspiranz-Wirkstoffes beobachtet, wenn die Zahlen m und p in den Estern der Formel (B1) der

Formel

$$m + 1 = p$$

entsprechen.

In einer weiteren ganz besonders bevorzugten Ausführungsform ist ein erfindungsgemäßer Antitranspirant-Stift daher dadurch gekennzeichnet, dass er einen Ester (B) der Formel (B1) enthält, in welcher die Zahlen m und p die folgende Gleichung erfüllen:

$$m + 1 = p$$

Besonders bevorzugte Ester (B) werden ausgewählt aus der Gruppe 1-Octansäure(1-octylester), 1-Nonansäure(1-nonylester), 1-Decansäure(1-decylester), 1-Undecansäure(1-undecylester), 1-Dodecansäure(1-dodecylester), 1-Tridecansäure(1-tridecylester) und 1-Tetradecansäure(1-tetradecylester (Alternativame: Myristylmyristat).

Explizit ganz besonders bevorzugt ist 1-Tetradecansäure-1-tetradecylester (Alternativame: Myristylmyristat, Tetradecyl Tetradecanoat) mit der CAS-Nummer 3234-85-3.

In einer weiteren Ausführungsform besonders bevorzugt ist ein Antitranspirant-Stift, enthaltend

(A) mindestens ein nichtionisches, polyalkoxyliertes Silikon
(B) 1-Tetradecansäure-1- tetradecylester (Myristylmyristat) und
(C) mindestens einen schweißhemmenden Wirkstoff aus der Gruppe der Aluminium- oder Aluminium-Zirkonium-Salze.

In einer weiteren Ausführungsform besonders bevorzugt ist ein Antitranspirant-Stift, enthaltend

(A) PEG-12 Dimethicon
(B) 1-Tetradecansäure-1- tetradecylester (Myristylmyristat) und
(C) mindestens einen schweißhemmenden Wirkstoff aus der Gruppe der Aluminium- oder Aluminium-Zirkonium-Salze.

Die erfindungsgemäßen Antitranspirant-Stifte werden wasserarm oder bevorzugt wasserfrei konfektioniert. Der Begriff "wasserarm" wird so verstanden, dass die Antitranspirant-Zusammensetzungen 2,5 bis maximal 10 Gew.-% freies Wasser enthalten, bezogen auf das Gesamtgewicht des Stiftes. Der Begriff "wasserfrei" wird erfindungsgemäß so verstanden, dass die Zusammensetzungen 0 bis maximal 2,5 Gew.-%, bevorzugt 0 bis maximal 1,0 Gew.-%, freies Wasser enthalten, bezogen auf das Gesamtgewicht des Stiftes. Der Gehalt an Kristallwasser, Hydratationswasser oder ähnlich molekular gebundenem Wasser, der in den eingesetzten Bestandteilen, insbesondere in gegebenenfalls enthaltenen schweißhemmenden Wirkstoffen, enthalten sein kann, stellt im Sinne der vorliegenden Anmeldung kein freies Wasser dar.

In einer weiteren ganz besonders bevorzugten Ausführungsform ist ein erfindungsgemäßer Antitranspirant-Stift daher dadurch gekennzeichnet, dass er einen Wassergehalt von maximal 10,0 Gew.-%, bevorzugt von maximal 7,5 Gew.-%, weiter bevorzugt von maximal 5,0 Gew.-%, noch weiter bevorzugt von maximal 2,5 Gew.-% und besonders bevorzugt von maximal 1,0 Gew.-% - bezogen auf das Gesamtgewicht des Stiftes - besitzt.

Unter dem Gesamtgewicht des Stiftes wird in diesem Zusammenhang das Gesamtgewicht der in Stift-, Kegel- bzw. Zylinderform vorliegenden Antitranspirant-Zusammensetzung (ohne Berücksichtigung jeglichen zur Verpackung oder zur Umhüllung verwendeten Materials) verstanden.

Zielsetzung der vorliegenden Erfindung ist die Minimierung bzw. Verhinderung der bei wiederholter Anwendung des Stiftes auftretenden Verkrustungen bzw. Verhärtungen. Auch nach wiederholter Anwendung soll der Stift noch eine cremige Konsistenz aufweisen. Dieses Ziel wird durch Zusatz einer Kombination aus alkoxy-modifiziertem Silikon (A) und Ester aus einer C8-C14-Alkansäure und einem C8-C14-Alkanol (B) zur Wachsmatrix des Stiftes erzielt.

Mit anderen Worten wird durch Zusatz der beiden Wirkstoffe (A) und (B) die Hydrophobizität der Wachsmatrix gerade so weit herabgesetzt, dass die schweißhemmenden Wirkstoffe (C) sich signifikant in der Wasserphase (d.h. dem Schweiß) zu lösen vermögen, ein übermäßiger Eintrag von Wasser in die Wachsmatrix aber gleichzeitig verhindert wird. Durch Zusatz der Wirkstoffe (A) und (B) wird die Hydrophobizität der Wachsmatrix demnach definiert auf den optimalen Wert eingestellt. Eine optimale Einstellung der Hydrophobizität ist insbesondere dann möglich, wenn die Wirkstoffe (A) und (B) in bestimmten Mengenbereichen eingesetzt werden.

Das Entstehen der durch die Anwendung des Stiftes hervorgerufenen Verhärtungen bzw. Verkrustungen konnte besonders dann reduziert oder gar verhindert werden, wenn ein oder mehrere alkoxy-modifizierte Silikone (A) in einer Gesamtmenge von 0,5 bis 10 Gew.-%, bevorzugt von 1,0 bis 8,0 Gew.-%, weiter bevorzugt von 1,8 bis 7,5 Gew.-% und besonders bevorzugt von 2,1 bis 5,2 Gew.-% - bezogen auf das Gesamtgewicht des Stiftes - eingesetzt wurden.

In einer weiteren ganz besonders bevorzugten Ausführungsform ist ein erfindungsgemäßer Antitranspirant-Stift daher dadurch gekennzeichnet, dass er ein oder mehrere alkoxy-modifizierte Silikone (A) in einer Gesamtmenge von 0,5 bis 10 Gew.-%, bevorzugt von 1,0 bis 8,0 Gew.-%, weiter bevorzugt von 1,8 bis 7,5 Gew.-% und besonders bevorzugt von 2,1 bis 5,2 Gew.-% - bezogen auf das Gesamtgewicht des Stiftes - enthält.

Weiterhin wurde war die cremige Konsistenz des Stiftes insbesondere dann auch noch nach wiederholter Anwendung vorhanden, wenn der Stift eine oder mehrere Ester (B) in einer Gesamtmenge von 0,5 bis 5,0 Gew.-%, bevorzugt von 1,0 bis 4,0 Gew.-%, weiter bevorzugt von 1,3 bis 3,5 Gew.-% und besonders bevorzugt von 1,6 bis 2,5 Gew.-% - bezogen auf das Gesamtgewicht des Stiftes - enthielt.

In einer weiteren ganz besonders bevorzugten Ausführungsform ist ein erfindungsgemäßer Antitranspirant-Stift daher dadurch gekennzeichnet, dass er eine oder mehrere Ester (B) in einer Gesamtmenge von 0,5 bis 5,0 Gew.-%, bevorzugt von 1,0 bis 4,0 Gew.-%, weiter bevorzugt von 1,3 bis 3,5 Gew.-% und besonders bevorzugt von 1,6 bis 2,5 Gew.-% - bezogen auf das Gesamtgewicht des Stiftes - enthält.

Unter dem Gesamtgewicht des Stiftes wird in diesem Zusammenhang wieder das Gesamtgewicht der in Stift-,

Kegel- bzw. Zylinderform vorliegenden Antitranspirant-Zusammensetzung (ohne Berücksichtigung jeglichen zur Verpackung oder zur Umhüllung verwendeten Materials) verstanden.

Bei der Einstellung der Hydrophobizität der Wachsmatrix wirken die Wirkstoffe der beiden Wirkstoffgruppen (A) und (B) zusammen und üben einen gegenseitigen Einfluss aufeinander aus. Hierbei hat es sich insbesondere als vorteilhaft herausgestellt, wenn das bzw. die Silikone (A) und der bzw. die Ester (B) entweder in gleichen Mengen eingesetzt werden, oder aber wenn das bzw. die Silikone (A) im Verhältnis zu dem bzw. den Estern (B) in einem Überschuss eingesetzt werden. Von besonderem Vorteil war es, wenn das Gewichtsverhältnis aus allen im Stift enthaltenen alkoxy-modifizierten Silikonen (A) zu allen im Stift enthaltenen Estern (B), d.h. das Gewichtsverhältnis (A)/(B), auf einen Wert von 1,0 bis 4,2, bevorzugt von 1,1 bis 4,0, weiter bevorzugt von 1,6 bis 3,2 und besonders bevorzugt von 1,8 bis 2,4 eingestellt wurde.

In einer weiteren ganz besonders bevorzugten Ausführungsform ist ein erfindungsgemäßer Antitranspirant-Stift daher dadurch gekennzeichnet, dass das Gewichtsverhältnis aus allen im Stift enthaltenen alkoxy-modifizierten Silikonen (A) zu allen im Stift enthaltenen Estern (B), d.h. das Gewichtsverhältnis (A)/(B), bei einem Wert von 1,0 bis 4,2, bevorzugt von 1,1 bis 4,0, weiter bevorzugt von 1,6 bis 3,2 und besonders bevorzugt von 1,8 bis 2,4 liegt.

Beispiel: Ein Antitranspirant Stift enthält

(A) 4,0 Gew.-% PEG-12 Dimethicon
(B) 1,9 Gew.-% Myristylmyristat
(C) 17,8 Gew.-% Aluminium Zirconium Trichlorhydrex Gly (AAZG 531 Summit Reheis)

weitere Inhaltsstoffe: ad 100 Gew.-%

Das Gewichtsverhältnis (A)/(B) liegt bei (4,0/1,9) = 2,1

Als dritten erfindungswesentlichen Inhaltsstoff (C) enthalten die Antitranspirant-Stifte der vorliegenden Erfindung mindestens einen schweißhemmenden Wirkstoff aus der Gruppe der Aluminium- oder Aluminium-Zirkonium-Salze.

Die schweißhemmenden Aluminiumsalze sind bevorzugt ausgewählt aus den wasserlöslichen adstringierenden anorganischen und organischen Salzen von Aluminium und Aluminium-Zirconium-Mischungen. Alumosilicate und Zeolithe zählen erfindungsgemäß nicht zu den Antitranspirant-Wirkstoffen. Erfindungsgemäß wird unter Wasserlöslichkeit eine Löslichkeit von wenigstens 3 Gew.-% bei 20 °C verstanden, das heißt, dass Mengen von wenigstens 3 g des Antitranspirant-Wirkstoffs in 97 g Wasser bei 20 °C löslich sind.

Besonders bevorzugte Antitranspirant-Wirkstoffe sind ausgewählt aus Aluminiumchlorhydrat, insbesondere Aluminiumchlorhydrat mit der allgemeinen Formel $[Al_2(OH)_5Cl \cdot 1\text{-}6\ H_2O]_n$, bevorzugt $[Al_2(OH)_5Cl \cdot 2\text{-}3\ H_2O]_n$, das in nicht-aktivierter oder in aktivierter (depolymerisierter) Form vorliegen kann, sowie Aluminiumchlorhydrat mit der allgemeinen Formel $[Al_2(OH)_4Cl_2 \cdot 1\text{-}6\ H_2O]_n$, bevorzugt $[Al_2(OH)_4Cl_2 \cdot 2\text{-}3\ H_2O]_n$, das in nicht-aktivierter oder in aktivierter (depolymerisierter) Form vorliegen kann.

Weiterhin bevorzugt sind Aluminiumsesquichlorhydrat, Aluminiumdichlorhydrat, Aluminiumchlorhydrex-Propylenglykol (PG) oder Aluminiumchlorhydrex-Polyethylenglykol (PEG), Aluminium- oder Aluminiumzirkonium-Glycol-Komplexe, z. B. Aluminium- oder Aluminiumzirkonium-Propylenglycol-Komplexe, Aluminiumsesquichlorhydrex-PG oder Aluminiumsesquichlorhydrex-PEG, Aluminium-PG-dichlorhydrex oder Aluminium-PEG-dichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirconium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat $(KAl(SO_4)_2 \cdot 12\ H_2O$, Alaun), Aluminiumundecylenoylcollagenaminosäure, Natriumaluminiumlactat + Aluminiumsulfat, Natriumaluminiumchlorhydroxylactat, Aluminiumbromhydrat, Aluminiumchlorid, den Aluminiumsalzen von Lipoaminosäuren, Aluminiumsulfat, Aluminiumlactat, Aluminiumchlorhydroxyallantoinat und Natrium-Aluminium-Chlorhydroxylactat. Erfindungsgemäß besonders bevorzugte Antitranspirant-Wirkstoffe sind ausgewählt aus so genannten "aktivierten" Aluminium- und Aluminium-Zirconiumsalzen, die auch als Antitranspirant-Wirkstoffe "mit erhöhter Wirksamkeit (englisch: enhanced activity)" bezeichnet werden. Derartige Wirkstoffe sind im Stand der Technik bekannt und auch kommerziell erhältlich. Aktivierte Aluminium- und Aluminium-Zirconiumsalze werden in der Regel durch Wärmebehandlung einer relativ verdünnten Lösung des Salzes erzeugt (z.B. etwa 10 Gew.-% Salz), um dessen HPLC-Peak 4-zu-Peak 3-Flächenverhältnis zu vergrößern. Das aktivierte Salz kann anschließend zu einem Pulver getrocknet, insbesondere sprühgetrocknet werden. Neben der Sprühtrocknung ist z. B. auch die Walzentrocknung geeignet.

Aktivierte Aluminium- und Aluminium-Zirconiumsalze haben typischerweise ein HPLC-Peak 4-zu-Peak 3-Flächenverhältnis von mindestens 0,4, bevorzugt mindestens 0,7, besonders bevorzugt mindestens 0,9, wobei mindestens 70% des Aluminiums diesen Peaks zuzuordnen sind.

Aktivierte Aluminium- und Aluminium-Zirconiumsalze müssen nicht notwendigerweise als sprühgetrocknetes Pulver eingesetzt werden. Erfindungsgemäß ebenfalls bevorzugte schweißhemmende Wirkstoffe sind nicht-wässrige Lösungen oder Solubilisate eines aktivierten schweißhemmenden Aluminium- oder Aluminium-Zirconiumsalzes, die

durch den Zusatz einer wirksamen Menge eines mehrwertigen Alkohols, der 3 bis 6 Kohlenstoffatome und 3 bis 6 Hydroxyl-Gruppen, bevorzugt Propylenglycol, Sorbit und Pentaerythrit, aufweist, gegen den Verlust der Aktivierung gegen den raschen Abbau des HPLC-Peak 4:Peak 3-Flächenverhältnisses des Salzes stabilisiert sind. Beispielsweise bevorzugt sind Zusammensetzungen, die in Gewichtsprozent (USP) enthalten: 18 - 45 Gew.-% eines aktivierten Aluminium- oder Aluminium-Zirconiumsalzes, 55 - 82 Gew.-% mindestens eines wasserfreien mehrwertigen Alkohols mit 3 bis 6 Kohlenstoffatomen und 3 bis 6 Hydroxyl-Gruppen, bevorzugt 1,2-Propylenglycol, 1,3-Butylenglycol, Diethylenglycol, Dipropylenglycol, Glycerin, Sorbit und Pentaerythrit, besonders bevorzugt 1,2-Propylenglycol.

Besonders bevorzugt sind auch Komplexe aktivierter schweißhemmender Aluminium- oder Aluminium-Zirconiumsalze mit einem mehrwertigen Alkohol, die 20 - 50 Gew.-%, besonders bevorzugt 20 - 42

Gew.-%, aktiviertes schweißhemmendes Aluminium- oder Aluminium-Zirconiumsalz und 2-16 Gew.-% molekular gebundenes Wasser enthalten, wobei der Rest zu 100 Gew.-% mindestens ein mehrwertiger Alkohol mit 3 bis 6 Kohlenstoffatome und 3 bis 6 Hydroxyl-Gruppen ist. Propylenglycol, Propylenglycol/Sorbit-Mischungen und Propylenglycol/Pentaerythrit-Mischungen sind bevorzugte derartige Alkohole.

Weitere bevorzugte schweißhemmende Wirkstoffe sind basische Calcium-Aluminiumsalze. Diese Salze werden durch Umsetzen von Calciumcarbonat mit Aluminiumchlorhydroxid oder Aluminiumchlorid und Aluminiumpulver oder durch Zusetzen von Calciumchlorid-Dihydrat zu Aluminiumchlorhydroxid hergestellt.

Weitere bevorzugte schweißhemmende Wirkstoffe sind Aluminium-Zirconium-Komplexe, die mit Salzen von Aminosäuren, insbesondere mit Alkali- und Erdalkaliglycinaten, gepuffert sind.

Weitere bevorzugte schweißhemmende Wirkstoffe sind aktivierte Aluminium- oder Aluminium-Zirconiumsalze, enthaltend 5 - 78 Gew.-% (USP) eines aktivierten schweißhemmenden Aluminium- oder Aluminium-Zirconiumsalzes, eine Aminosäure oder Hydroxyalkansäure in einer solchen Menge, um ein (Aminosäure oder Hydroxyalkansäure) zu (Al+Zr) - Gewichtsverhältnis von 2:1 - 1:20 und bevorzugt 1:1 bis 1:10 bereitzustellen, sowie ein wasserlösliches Calciumsalz in einer solchen Menge, um ein Ca:(Al+Zr)-Gewichtsverhältnis von 1:1 - 1:28 und bevorzugt 1:2 - 1:25 bereitzustellen. Besonders bevorzugte feste aktivierte schweißhemmende Salzzusammensetzungen enthalten 48 - 78 Gew.-% (USP), bevorzugt 66 - 75 Gew.-% eines aktivierten Aluminium- oder Aluminium-Zirconiumsalzes und 1 - 16 Gew.-%, bevorzugt 4 - 13 Gew.-% molekular gebundenes Wasser (Hydratationswasser), weiterhin so viel wasserlösliches Calciumsalz, dass das Ca:(Al+Zr)-Gewichtsverhältnis 1:1 - 1:28, bevorzugt 1:2 - 1:25, beträgt und s oviel Aminosäure, dass das Aminosäure zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48 - 78 Gew.-% (USP), bevorzugt 66 - 75 Gew.-% eines aktivierten Aluminium- oder Aluminium-Zirconiumsalzes und 1-16 Gew.-%, bevorzugt 4 - 13 Gew.-% molekular gebundenes Wasser (Hydratationswasser), weiterhin soviel wasserlösliches Calciumsalz, dass das Ca:(Al+Zr)-Gewichtsverhältnis 1:1 - 1:28, bevorzugt 1:2 - 1:25, beträgt und soviel Glycin, dass das Glycin zu (Al+Zr) - Gewichtsverhältnis 2:1 - 1:20, bevorzugt 1:1 - 1:10, beträgt.

Weitere besonders bevorzugte feste schweißhemmende aktivierte Salzzusammensetzungen enthalten 48 - 78 Gew.-% (USP), bevorzugt 66 - 75 Gew.-% eines aktivierten Aluminium- oder Aluminium-Zirconiumsalzes und 1-16 Gew.-%, bevorzugt 4 - 13 Gew.-% molekular gebundenes Wasser, weiterhin soviel wasserlösliches Calciumsalz, dass das Ca:(Al+Zr)-Gewichtsverhältnis 1:1 - 1:28, bevorzugt 1:2 - 1:25, beträgt und soviel Hydroxyalkansäure, dass das Hydroxyalkansäure zu (Al+Zr) - Gewichtsverhältnis 2:1 - 1:20, bevorzugt 1:1 - 1:10, beträgt.

Für die Stabilisierung der schweißhemmenden Salze bevorzugte Aminosäuren sind ausgewählt aus Glycin, Alanin, Leucin, Isoleucin, β-Alanin, Valin, Cystein, Serin, Tryptophan, Phenylalanin, Methionin, β-Amino-n-butansäure und γ-Amino-n-butansäure und den Salzen davon, jeweils in der d-Form, der l-Form und der dl-Form; Glycin ist besonders bevorzugt.

Für die Stabilisierung der schweißhemmenden Salze bevorzugte Hydroxyalkansäuren sind ausgewählt aus Glycolsäure und Milchsäure.

Weitere bevorzugte aktivierte Aluminiumsalze sind solche der allgemeinen Formel $Al_2(OH)_{6-a}Xa$, worin X Cl, Br, I oder $NO_3$ ist und "a" ein Wert von 0,3 bis 5, bevorzugt von 0,8 bis 2,5 und besonders bevorzugt 1 bis 2 ist, so dass das Molverhältnis von Al:X 0,9:1 bis 2,1:1 beträgt. Bei diesen Salzen ist im Allgemeinen etwas Hydratationswasser assoziativ gebunden, typischerweise 1 bis 6 Mol Wasser pro Mol Salz. Besonders bevorzugt ist Aluminiumchlorhydrat (d.h. X ist Cl in der vorgenannten Formel) und speziell 5/6-basisches Aluminiumchlorhydrat, worin "a" 1 beträgt, so dass das Molverhältnis von Aluminium zu Chlor 1,9:1 bis 2,1:1 beträgt.

Bevorzugte aktivierte Aluminium-Zirconiumsalze sind solche, die Mischungen oder Komplexe der vorstehend beschriebenen Aluminiumsalze mit Zirconiumsalzen der Formel $ZrO(OH)_{2-pb}Y_b$ darstellen, worin Y Cl, Br, I, $NO_3$ oder $SO_4$ ist, b eine rationale Zahl von 0,8 bis 2 und p die Wertigkeit von Y ist, wie sie beispielsweise in US 6074632 offenbart sind. Die Zirconiumsalze haben in der Regel ebenfalls etwas Hydratationswasser assoziativ gebunden, typischerweise 1 bis 7 Mol Wasser pro Mol Salz. Vorzugsweise ist das Zirconiumsalz Zirconylhydroxychlorid mit der Formel $ZrO(OH)_{2-b}Cl_b$, worin b eine rationale Zahl von 0,8 bis 2, bevorzugt 1,0 bis 1,9 ist. Bevorzugte Aluminium-Zirconiumsalze haben ein Al:Zr-Molverhältnis von 2 bis 10 und ein Metall:(X+Y)-Verhältnis von 0,73 bis 2,1, bevorzugt 0,9 bis 1,5. Ein besonders bevorzugtes Salz ist Aluminium-Zirconiumchlorhydrat (d.h., X und Y sind Cl), das ein Al:Zr-Verhältnis von 2

bis 10 und ein molares Metall:Cl-Verhältnis von 0,9 bis 2,1 hat. Der Begriff Aluminium-Zirconiumchlorhydrat umfasst die Tri-, Tetra-, Penta- und Octachlorhydratformen. Bevorzugte schweißhemmende Aluminium-Zirconium-Salze weisen ein molares Metall-zu-Chlorid-Verhältnis von 0,9 - 1,5, bevorzugt 0,9 - 1,3, besonders bevorzugt 0,9 - 1,1, auf.

Erfindungsgemäß besonders bevorzugte Zirconium-freie Aluminiumsalze weisen ein molares Metall-zu-Chlorid-Verhältnis von 1,9 - 2,1 auf. Erfindungsgemäß besonders bevorzugte Zirconium-freie Aluminiumsesquichlorohydrate weisen ein molares Metall-zu-Chlorid-Verhältnis von 1,5:1-1,8:1 auf. Bevorzugte Aluminiumzirconiumchlorohydrate haben im allgemeinen die empirische Formel $Al_nZr(OH)_{[3n+4-m(n+1)]}(Cl)_{[m(n+1)]}$ mit n = 2,0 - 10,0, bevorzugt 3,0 - 8,0, m = 0,77 - 1,11 (entsprechend einem molaren Metall (Al+Zr)-zu-Chlorid-Verhältnis von 1,3 - 0,9), bevorzugt m = 0,91 - 1,11 (entsprechend M:Cl = 1,1 - 0,9), und besonders bevorzugt m = 1,00 - 1,11 (entsprechend M:Cl = 1,0 - 0,9), weiterhin sehr bevorzugt m = 1,02 - 1,11 (entsprechend M:Cl = 0,98 - 0,9) sowie sehr bevorzugt m = 1,04 - 1,11 (entsprechend M:Cl = 0,96 - 0,9). Bei diesen Salzen ist im Allgemeinen etwas Hydratationswasser assoziativ gebunden, typischerweise 1 - 6 Mol Wasser pro Mol Salz, entsprechend 1-16 Gew.-%, bevorzugt 4 - 13 Gew.-% Hydratationswasser.

Üblicherweise sind die bevorzugten Aluminiumzirconiumchlorohydrate mit einer Aminosäure assoziiert, um die Polymerisation der Zirconiumspecies während der Herstellung zu verhindern. Bevorzugte stabilisierende Aminosäuren sind ausgewählt aus Glycin, Alanin, Leucin, Isoleucin, β-Alanin, Cystein, Valin, Serin, Tryptophan, Phenylalanin, Methionin, β-Amino-n-butansäure und γ-Amino-n-butansäure und den Salzen davon, jeweils in der d-Form, der l-Form und der dl-Form; Glycin ist besonders bevorzugt. Die Aminosäure ist in einer Menge von 1 - 3 Mol, bevorzugt 1,3 - 1,8 Mol, jeweils pro Mol Zirconium in dem Salz enthalten.

Bevorzugte schweißhemmende Salze sind Aluminium-Zirconiumtetrachlorohydrate (Al:Zr = 2-6; M:Cl = 0.9-1.3), insbesondere Salze mit einem molaren Metall-zu-Chlorid-Verhältnis von 0,9 - 1,1, bevorzugt 0,9-1,0.

Weiterhin erfindungsgemäß bevorzugt sind Aluminiumzirconiumchlorohydrat-Glycin-Salze, die mit Betain ($(CH_3)_3N^+-CH_2-COO^-$) stabilisiert sind. Besonders bevorzugte entsprechende Verbindungen weisen ein molares Gesamt-(Betain + Glycin)/Zr-Verhältnis von (0,1 - 3,0) : 1, bevorzugt (0,7 - 1,5) : 1 und ein molares Verhältnis von Betain zu Glycin von mindestens 0,001 : 1 auf.

In einer besonders bevorzugten erfindungsgemäßen Ausführungsform ist als besonders wirksames Antitranspirant-Salz ein so genanntes "aktiviertes" Salz enthalten, insbesondere eines mit einem hohen HPLC-Peak 5-Aluminium-Gehalt, insbesondere mit einer Peak 5-Fläche von mindestens 33 %, besonders bevorzugt mindestens 45 %, bezogen auf die gesamte Fläche unter den Peaks 2 - 5, gemessen mit HPLC einer 10 Gew.-%igen wässrigen Lösung des Wirkstoffs unter Bedingungen, bei denen die Aluminiumspecies in mindestens 4 aufeinander folgende Peaks aufgelöst werden (mit Peaks 2 - 5 bezeichnet). Bevorzugt sind Aluminiumzirconiumsalze mit einem hohen HPLC-Peak 5-Aluminium-Gehalt (auch als "$E^5$AZCH" bezeichnet).

Weiterhin sind solche aktivierten "$E^5$AZCH"-Salze bevorzugt, deren HPLC-Peak 4-zu-Peak 3-Flächenverhältnis von mindestens 0,4, bevorzugt mindestens 0,7, besonders bevorzugt mindestens 0,9, beträgt. Weitere besonders bevorzugte Antitranspirant-Wirkstoffe sind solche Aluminiumzirconiumsalze mit einem hohen HPLC-Peak 5-Aluminium-Gehalt, die zusätzlich mit einem wasserlöslichen Strontiumsalz und/oder mit einem wasserlöslichen Calciumsalz stabilisiert sind.

Die Formulierung der erfindungsgemäßen Mittel in einer bestimmten Darreichungsform, wie beispielsweise einem Antitranspirant-Rollon oder einem Antitranspirantstift, Antitranspirantgel richtet sich bevorzugt nach den Anforderungen des Verwendungszwecks.

Der Antitranspirant Stift kann den bzw. die schweißhemmenden Wirkstoffe - insbesondere die vorgenannten bevorzugten und besonders bevorzugten Vertreter - (C) in einer Gesamtmenge von 5,0 bis 20,0 Gew.-%, bevorzugt von 7,0 bis 17,5 Gew.-%, weiter bevorzugt von 8,5 bis 15,5 Gew.-% und besonders bevorzugt von 11,5 bis 14,5 Gew.-% - bezogen auf das Gesamtgewicht des Stiftes - enthalten.

In einer weiteren ganz besonders bevorzugten Ausführungsform ist ein erfindungsgemäßer Antitranspirant-Stift daher dadurch gekennzeichnet, dass er einen oder mehrere schweißhemmende Wirkstoffe (C) in einer Gesamtmenge von 5,0 bis 20,0 Gew.-%, bevorzugt von 7,0 bis 17,5 Gew.-%, weiter bevorzugt von 8,5 bis 15,5 Gew.-% und besonders bevorzugt von 11,5 bis 14,5 Gew.-% - bezogen auf das Gesamtgewicht des Stiftes - enthält.

Für eine optimierte Wirkstofffreisetzung werden auch das bzw. die schweißhemmenden Wirkstoffe (C) zur Summe aus alkoxy-modifizierten Silikonen (A) und Estern (B) in einem bestimmten Verhältnis zueinander eingesetzt. Eine besonders gute Schweißreduktion konnte beobachtet werden, wenn das Gewichtsverhältnis aus allen im Stift enthaltenen schweißhemmenden Wirkstoffen (C) zur Summe der im Stift enthaltenen alkoxy-modifizierten Silikone (A) und Ester (B), d.h. das Gewichtsverhältnis (C)/[(A)+(B)], bei einem Wert von 1,0 bis 4,5, bevorzugt von 1,5 bis 3,5, weiter bevorzugt von 1,8 bis 3,1 und besonders bevorzugt von 1,9 bis 2,5 lag.

In einer weiteren ganz besonders bevorzugten Ausführungsform ist ein erfindungsgemäßer Antitranspirant-Stift daher dadurch gekennzeichnet, dass das Gewichtsverhältnis aus allen im Stift enthaltenen schweißhemmenden Wirkstoffen (C) zur Summe der im Stift enthaltenen alkoxy-modifizierten Silikone (A) und Ester (B), d.h. das Gewichtsverhältnis (C)/[(A)+(B)], bei einem Wert von 1,0 bis 4,5, bevorzugt von 1,5 bis 3,5, weiter bevorzugt von 1,8 bis 3,1 und besonders bevorzugt von 1,9 bis 2,5 liegt.

Beispiel: Ein Antitranspirant Stift enthält

(A) 4,0 Gew.-% PEG-12 Dimethicone
(B) 1,9 Gew.-% Myristylmyristat
(C) 17,8 Gew.-% Aluminium Zirconium Trichlorhydrex Gly (Summit Reheis) (Gehalt Aktivsubstanz = 78,5 %, Gehalt Aktivsubstanz = 13,97 Gew.-%)

weitere Inhaltsstoffe: ad 100 Gew-%
Das Gewichtsverhältnis (C)/[(A)+(B)] = [13,97 / (1,9 + 4,0)] = 2,37

Überraschenderweise konnte der Einsatz eines oder mehrere Polyethylenglycole die Konsistenz der Antitranspirant-Stifte noch weiter verbessern.

Unter Polyethylenglycol wird eine Verbindung der allgemeinen Formel (D) verstanden

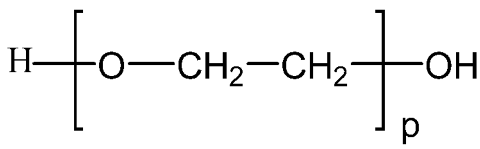
(D),

wobei p eine ganze Zahl von 2 bis 500, bevorzgut von 2 bis 250, weiter bevorzugt von 2 bis 200 und besonders bevorzugt von 2 bis 150 darstellt. Das bzw. die Polyethylenglycole sind im Antitranspirant-Stift bevorzugt in einer Gesamtmenge von 0,01 bis 10,0 Gew.-%, bevorzugt von 0,07 bis 7,0 Gew.-%, weiter bevorzugt von 0,15 bis 4,5 Gew.-% und besonders bevorzugt von 0,25 bis 1,0 Gew.-% - bezogen auf das Gesamtgewicht des Stiftes - enthalten.

In einer weiteren ganz besonders bevorzugten Ausführungsform ist ein erfindungsgemäßer Antitranspirant-Stift daher dadurch gekennzeichnet, dass er zusätzlich ein oder mehrere Polyethylenglycole in einer Gesamtmenge von 0,01 bis 10,0 Gew.-%, bevorzugt von 0,07 bis 7,0 Gew.-%, weiter bevorzugt von 0,15 bis 4,5 Gew.-% und besonders bevorzugt von 0,25 bis 1,0 Gew.-% - bezogen auf das Gesamtgewicht des Stiftes - enthält.

Die erfindungsgemäßen Antitranspirantstifte enthalten die erfindungswesentlichen Wirkstoffe (A), (B) und (C) bevorzugt in einer Lipid- oder Wachsmatrix. Die Lipid- oder Wachsmatrix der erfindungsgemäßen Stiftzusammensetzungen kann mindestens eine Lipid- oder Wachskomponente mit einem Schmelzpunkt > 50°C umfassen. Generell sind Wachse von fester bis brüchig harter Konsistenz, grob bis feinkristallin, durchscheinend bis opak, jedoch nicht glasartig, und schmelzen oberhalb von 50 °C ohne Zersetzung. Sie sind schon wenig oberhalb des Schmelzpunktes niedrigviskos und zeigen eine stark temperaturabhängige Konsistenz und Löslichkeit.

Erfindungsgemäß bevorzugt sind beispielsweise natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse und Polyethylenglycolwachse, $C_{20}$-$C_{40}$-Dialkylester von Dimersäuren, $C_{30-50}$-Alkylbienenwachs sowie Alkyl- und Alkylarylester von Dimerfettsäuren.

Eine Wachskomponente, die zusätzlich zu den erfindungsgemäßen Estern (B) im Antitranspirant-Stift enthalten sein kann, ist ausgewählt aus mindestens einem Ester aus einem gesättigten, einwertigen $C_{16}$-$C_{60}$-Alkohol und einer gesättigten $C_{16}$-$C_{36}$-Monocarbonsäure.

Erfindungsgemäß zählen hierzu auch Lactide, die cyclischen Doppelester von $\alpha$-Hydroxycarbonsäuren der entsprechenden Kettenlänge. Die Ester setzen sich aus gesättigten, verzweigten oder unverzweigten Monocarbonsäuren und gesättigten, verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten, verzweigten oder unverzweigten Alkoholen sind erfindungsgemäß einsetzbar, sofern die Wachskomponente einen Schmelzpunkt > 50°C hat.

Diese Wachskomponenten können aus der Gruppe der Ester aus gesättigten, verzweigten oder unverzweigten Alkancarbonsäuren einer Kettenlänge von 16 bis 24 C-Atomen und den gesättigten, verzweigten oder unverzweigten Alkoholen einer Kettenlänge von 16 bis 50 C-Atomen, die einen Schmelzpunkt > 50°C haben, ausgewählt werden.

Insbesondere können als Wachskomponente $C_{16-36}$-Alkylstearate und $C_{18-38}$-Alkylhydro-xystearoylstearate, $C_{20-40}$-Alkylerucate sowie Cetearylbehenat vorteilhaft sein. Das Wachs oder die Wachskomponenten weisen einen Schmelzpunkt > 50°C, bevorzugt > 60°C, auf.

Eine besonders bevorzugte Ausführungsform der Erfindung enthält als Wachskomponente ein $C_{20}$-$C_{40}$-Alkylstearat. Dieser Ester ist unter den Namen Kesterwachs® K82H oder Kesterwachs® K80H bekannt und wird von Koster Keunen Inc. vertrieben. Es handelt sich um die synthetische Nachahmung der Monoesterfraktion des Bienenwachses

und zeichnet sich durch seine Härte, seine Ölgelierfähigkeit und seine breite Kompatibiltät mit Lipidkomponenten aus. Dieses Wachs kann als Stabilisator und Konsistenzregulator für W/O- und O/W-Emulsionen verwendet werden. Kesterwachs bietet den Vorteil, dass es auch bei geringen Konzentrationen eine exzellente Ölgelierfähigkeit aufweist und so die Stiftmasse nicht zu schwer macht und einen samtigen Abrieb ermöglicht. Eine weitere besonders bevorzugte Ausführungsform der Erfindung enthält als Wachskomponente Cetearylbehenat, d. h. Mischungen aus Cetylbehenat und Stearylbehenat. Dieser Ester ist unter dem Namen Kesterwachs® K62 bekannt und wird von Koster Keunen Inc. vertrieben.

Weitere bevorzugte Lipid- oder Wachskomponenten mit einem Schmelzpunkt > 50°C sind die Triglyceride gesättigter und gegebenenfalls hydroxylierter $C_{12-30}$-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50 °C aufweisen, beispielsweise bevorzugt $C_{18}$ - $C_{36}$ Acid Triglyceride (Syncrowax® HGL-C). Erfindungsgemäß ist als Wachskomponente hydriertes Rizinusöl, erhältlich z. B. als Handelsprodukt Cutina® HR, besonders bevorzugt. Weitere bevorzugte Lipid- oder Wachskomponenten mit einem Schmelzpunkt > 50°C sind die gesättigten linearen $C_{14}$ - $C_{36}$-Carbonsäuren, insbesondere Myristinsäure, Palmitinsäure, Stearinsäure und Behensäure sowie Mischungen dieser Verbindungen, z. B. Syncrowax® AW 1C ($C_{18}$ - $C_{36}$-Fettsäuren) oder Cutina® FS 45 (Palmitin- und Stearinsäure).

Als festigende Fett- oder Wachskomponente eignen sich mehr oder weniger alle gesättigten, linearen Fettsäuren und Fettalkohole mit 14 - 22 C-Atomen, Triglyceride gesättigter Fettsäuren mit 14 - 22 C-Atomen, gesättigte lineare Wachsester (Fettsäure-Fettalkohol-Ester) mit 28 - 44 C-Atomen oder Gemische davon. Ganz besonders gut eignet sich eine Mischung aus 1 Gewichtsteil hydriertem Rizinusöl und 2 - 4 Gewichtsteilen Stearylalkohol.

Die Antitranspirant Stifte können zusätzlich auch noch weitere Emulgatoren wie beispielsweise Öl-in-Wasser-Emulgatoren enthalten.

Besonders bevorzugt ist der nichtionische Öl-in-Wasser-Emulgator ausgewählt ist aus grenzflächenaktiven Substanzen mit einem HLB-Wert von mehr als 7, ausgewählt aus :

- ethoxylierten $C_8$-$C_{24}$-Alkanolen mit durchschnittlich 10- 100 Mol Ethylenoxid pro Mol,
- ethoxylierten $C_8$-$C_{24}$-Carbonsäuren mit durchschnittlich 10-100 Mol Ethylenoxid pro Mol,
- Alkylmono- und -oligoglycosiden mit 8 bis 22 Kohlenstoffatomen im Alkylrest und deren ethoxylierten Analoga,
- ethoxylierten Sterinen,
- Partialestern von Polyglycerinen mit n = 2 bis 10 Glycerineinheiten und mit 1 bis 4 gesättigten oder ungesättigten, linearen oder verzweigten, gegebenenfalls hydroxylierten $C_8$ - $C_{30}$-Fettsäureresten verestert, sofern sie einen HLB-Wert von mehr als 7 aufweisen,
- sowie Mischungen der vorgenannten Substanzen.

Bevorzugte erfindungsgemäße Antitranspirant-Stifte sind dadurch gekennzeichnet, dass die nichtionischen Öl-in-Wasser-Emulgatoren ausgewählt sind aus ethoxylierten $C_8$-$C_{24}$-Alkanolen mit durchschnittlich 10 - 100 Mol Ethylenoxid pro Mol, ethoxylierten $C_8$-$C_{24}$-Carbonsäuren mit durchschnittlich 10 - 100 Mol Ethylenoxid pro Mol, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit n = 2 bis 10 Glycerineinheiten und mit 1 bis 4 gesättigten oder ungesättigten, linearen oder verzweigten, gegebenenfalls hydroxylierten $C_8$ - $C_{30}$-Fettsäureresten verestert, sofern sie einen HLB-Wert von mehr als 7 aufweisen, sowie Mischungen der vorgenannten Substanzen.

Die ethoxylierten $C_8$-$C_{24}$-Alkanole haben die Formel $R^1O(CH_2CH_2O)_nH$, wobei $R^1$ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.

Die ethoxylierten $C_8$-$C_{24}$-Carbonsäuren haben die Formel $R^1O(CH_2CH_2O)_nH$, wobei $R^1O$ steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat,

PEG-50-monolaurat und PEG-100-monolaurat.

Besonders bevorzugt eingesetzt werden die $C_{12}$-$C_{18}$-Alkanole oder die $C_{12}$-$C_{18}$-Carbonsäuren mit jeweils 10 - 30 Einheiten Ethylenoxid pro Molekül sowie Mischungen dieser Substanzen, insbesondere Ceteth-12, Ceteth-20, Ceteth-30, Steareth-12, Steareth-20, Steareth-30, Laureth-12 und Beheneth-20. Weiterhin werden vorzugsweise $C_8$-$C_{22}$-Alkylmono-und-oligoglycoside eingesetzt. $C_8$-$C_{22}$-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare® erhältlich sind, enthalten eine glucosidisch gebundene $C_8$-$C_{16}$-Alkylgruppe an einem Oligoglucosidrest, dessen mittlerer Oligomerisationsgrad bei 1 - 2 liegt. Besonders bevorzugte $C_8$-$C_{22}$-Alkylmono-und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten $C_8$-$C_{30}$-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Die Antitranspirant Stifte können zusätzlich auch noch weitere flüchtige Silikonöle und flüchtige Nichtsilikonöle enthalten.

Besonders bevorzugte flüchtige Öle sind flüchtige Silikonöle und flüchtige Nichtsilikonöle. Flüchtige Siliconöle, die cyclisch sein können, wie z. B. Octamethylcyclotetrasiloxan, Decamethylcyclopentasiloxan sowie Mischungen hiervon, wie sie z. B. in den Handelsprodukten DC 244, 245, 344 und 345 von Dow Corning enthalten sind, sind erfindungsgemäß bevorzugt. Ebenfalls besonders bevorzugt sind flüchtige lineare Siliconöle, insbesondere Hexamethyldisiloxan ($L_2$), Octamethyltrisiloxan ($L_3$), Decamethyltetrasiloxan ($L_4$) sowie beliebige Zweier- und Dreiermischungen aus $L_2$, $L_3$ und/oder $L_4$, bevorzugt solche Mischungen, wie sie z. B. in den Handelsprodukten DC 2-1184, Dow Corning® 200 (0,65 cSt) und Dow Corning® 200 (1,5 cSt) von Dow Corning enthalten sind.

Flüchtige Siliconöle sind erfindungsgemäß hervorragend geeignet, da sie der erfindungsgemäßen Zusammensetzung ein angenehmes Hautgefühl und eine geringe Kleideranschmutzung verleihen. Erfindungsgemäß besonders bevorzugte Zusammensetzungen sind daher durch einen Gehalt an mindestens einem flüchtigen Siliconöl gekennzeichnet. Dabei ist es wiederum bevorzugt, wenn die flüchtigen Silikonöle bezogen auf das Gewicht der Zusammensetzung in einer Gesamtmenge von 20 bis 80 Gew.-%, insbesondere von 30 bis 80 Gew.-%, besonders bevorzugt von 40 bis 75 Gew.-%, ganz besonders bevorzugt von 45 bis 70 Gew.-%, in der erfindungsgemäßen Zusammensetzung enthalten sind.

Neben oder anstelle des mindestens einen flüchtigen Siliconöls kann auch mindestens ein flüchtiges Nichtsiliconöl enthalten sein. Bevorzugte flüchtige Nichtsiliconöle sind ausgewählt aus $C_8$-$C_{16}$-Isoparaffinen, insbesondere aus Isodecan, Isododecan, Isotetradecan und Isohexadecan sowie Mischungen hiervon. Insbesondere eignet sich bevorzugt C10-13 Isoparaffin (z.B. Handelsprodukt Pioner 2094 von Hansen & Rosenthal).

Auch dieses mindestens eine flüchtige Nichtsiliconöl ist bevorzugt in einer Gesamtmenge von von 20 bis 80 Gew.-%, besonders bevorzugt von 30 bis 80 Gew.-%, ganz besonders bevorzugt von 40 bis 75 Gew.-%, jeweils bezogen auf das Gesamtgewicht der Zusammensetzung, enthalten.

Aufgrund des Hautgefühls und der Stabilität der resultierenden Zusammensetzungen sind Siliconöle als flüchtiges Öl gegenüber Isoparaffinen besonders bevorzugt.

Neben den vorgenannten, üblicherweise als flüchtigen Siliconölen bezeichneten Substanzen sowie neben den vorgenannten flüchtigen Nichtsiliconölen können die erfindungsgemäßen Zusammensetzungen zusätzlich mindestens

ein nichtflüchtiges Öl, ausgewählt aus nichtflüchtigen Siliconölen und nichtflüchtigen Nichtsiliconölen, enthalten.

Bevorzugte nichtflüchtige Siliconöle sind ausgewählt aus höhermolekularen linearen Dimethylpolysiloxanen, im Handel erhältlich z. B. unter der Bezeichnung Dow Corning® 190, Dow Corning® 200 Fluid mit kinematischen Viskositäten (25°C) im Bereich von 5 - 100 cSt, bevorzugt 5-50 cSt oder auch 5-10 cSt, und Baysilon® 350 M (mit einer kinematischen Viskosität (25°C) von etwa 350 cSt. Erfindungsgemäß ebenfalls bevorzugte nichtflüchtige Siliconöle sind ausgewählt aus Siliconen der Formel (Sil-1), wobei x ausgewählt ist aus ganzen Zahlen von 1 - 20, bevorzugt 1 - 3.

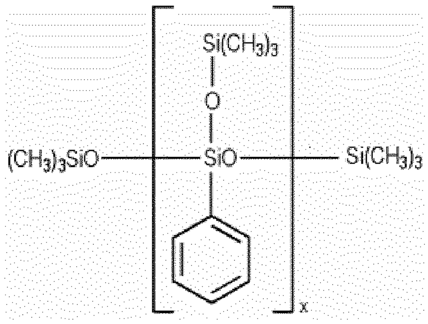

(Sil-1)

Ein bevorzugtes Siliconöl der Formel (Sil-1) ist erhältlich unter der INCI-Bezeichnung Phenyl Trimethicone.

Natürliche und synthetische Kohlenwasserstoffe, wie beispielsweise Paraffinöle, $C_{18}$-$C_{30}$-Isoparaffine, insbesondere Isoeicosan, Polyisobutene oder Polydecene, die beispielsweise unter der Bezeichnung Emery® 3004, 3006, 3010 oder unter der Bezeichnung Ethylflo® von Albemarle oder Nexbase® 2004G von Nestle erhältlich sind, sowie 1,3-Di-(2-ethylhexyl)-cyclohexan (erhältlich z. B. unter dem Handelsnamen Cetiol®S von Cognis) gehören ebenfalls zu den erfindungsgemäß bevorzugten nichtflüchtigen Nichtsiliconölen.

Weitere erfindungsgemäß bevorzugte nichtflüchtige Nichtsiliconöle sind ausgewählt aus den Benzoesäureestern von linearen oder verzweigten $C_{8-22}$-Alkanolen. Besonders bevorzugt sind Benzoesäure-C12-C15-alkylester, z. B. erhältlich als Handelsprodukt Finsolv® TN, Benzoesäureisostearylester, z. B. erhältlich als Handelsprodukt Finsolv® SB, Ethylhexylbenzoat, z. B. erhältlich als Handelsprodukt Finsolv® EB, und Benzoesäureoctyldocecylester, z. B. erhältlich als Handelsprodukt Finsolv® BOD.

Weitere erfindungsgemäß bevorzugte nichtflüchtige Nichtsiliconöle sind ausgewählt aus den Triglyceriden von linearen oder verzweigten, gesättigten oder ungesättigten, gegebenenfalls hydroxylierten $C_{8-30}$-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol® 318, Myritol® 331 (Cognis) oder Miglyol® 812 (Hüls) mit unverzweigten Fettsäureresten sowie Glyceryltriisostearin und die Handelsprodukte Estol® GTEH 3609 (Uniqema) oder Myritol® GTEH (Cognis) mit verzweigten Fettsäureresten.

Weitere erfindungsgemäß besonders bevorzugte nichtflüchtige Nichtsiliconöle sind ausgewählt aus den Dicarbonsäureestern von linearen oder verzweigten $C_2$-$C_{10}$-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß besonders bevorzugte nichtflüchtige Nichtsiliconöle sind ausgewählt aus den Anlagerungsprodukten von 1 bis 5 Propylenoxid-Einheiten an ein- oder mehrwertige $C_{8-22}$-Alkanole wie Octanol, Decanol, Decandiol, Laurylalkohol, Myristylalkohol und Stearylalkohol, z. B. PPG-2-Myristylether und PPG-3-Myristylether (Witconol® APM).

Weitere erfindungsgemäß besonders bevorzugte nichtflüchtige Nichtsiliconöle sind ausgewählt aus den Anlagerungsprodukten von mindestens 6 Ethylenoxid- und/oder Propylenoxid-Einheiten an ein- oder mehrwertige $C_{3-22}$-Alkanole wie Glycerin, Butanol, Butandiol, Myristylalkohol und Stearylalkohol, die gewünschtenfalls verestert sein können, z. B. PPG-14-Butylether (Ucon Fluid® AP), PPG-9-Butylether (Breox® B25), PPG-10-Butandiol (Macol® 57), PPG-15-Stearylether (Arlamol® E) und Glycereth-7-diisononanoat.

Weitere erfindungsgemäß besonders bevorzugte nichtflüchtige Nichtsiliconöle sind ausgewählt aus den $C_8$-$C_{22}$-Alkanolestern einwertiger oder mehrwertiger $C_2$-$C_7$-Hydroxycarbonsäuren, insbesondere die Ester der Glycolsäure, Milchsäure, Äpfelsäure, Weinsäure, Citronensäure und Salicylsäure. Solche Ester auf Basis von linearen $C_{14/15}$-Alkanolen, z. B. $C_{12}$-$C_{15}$-Alkyllactat, und von in 2-Position verzweigten $C_{12/13}$-Alkanolen sind unter dem Warenzeichen Cosmacol® von der Firma Nordmann, Rassmann GmbH & Co, Hamburg, zu beziehen, insbesondere die Handelsprodukte Cosmacol® ESI, Cosmacol® EMI und Cosmacol® ETI.

Weitere erfindungsgemäß besonders bevorzugte nichtflüchtige Nichtsiliconöle sind ausgewählt aus den symmetrischen, unsymmetrischen oder cyclischen Estern der Kohlensäure mit Alkanolen, z. B. Glycerincarbonat, Dicaprylylcarbonat (Cetiol® CC) oder die Ester gemäß der Lehre der DE 19756454 A1.

Weitere Öle, die erfindungsgemäß bevorzugt sein können, sind ausgewählt aus den Estern von Dimeren ungesättigter $C_{12}$-$C_{22}$-Fettsäuren (Dimerfettsäuren) mit einwertigen linearen, verzweigten oder cyclischen $C_2$-$C_{18}$-Alkanolen oder mit mehrwertigen linearen oder verzweigten $C_2$-$C_6$-Alkanolen.

Es ist erfindungsgemäß bevorzugt, wenn die erfindungsgemäßen Zusammensetzungen bezogen auf das Gesamtgewicht der Zusammensetzung die nichtflüchtigen Öle in einer Gesamtmenge von 0 bis 20 Gew.-%, insbesondere von 0 bis 10 Gew.-% enthalten.

Die erfindungsgemäße Zusammensetzung enthält besonders bevorzugt zusätzlich mindestens einen Fettalkohol. Unter Fettalkoholen werden erfindungsgemäß primäre Alkohole in Form von Monohydroxyverbindungen eines linearen, aliphatischen Kohlenwasserstoffs verstanden, wobei der besagte Kohlenwasserstoff wenigstens 12 Kohlenstoffatome aufweist und außer der Hydroxygruppe keinen weiteren Substituenten trägt.

Es ist erfindungsgemäß bevorzugt, wenn die zur Herstellung der erfindungsgemäßen Zusammensetzung verwendeten Fettalkohole als Feststoff vorliegen und sich alle auf Fettalkohole beziehenden Definitionen auf feste Fettalkohole im Sinne der Anmeldung beziehen und flüssige Fettalkohole ausschließlich der Ölkomponente der erfindungsgemäßen Zusammensetzung zugerechnet werden.

Bevorzugte erfindungsgemäße Zusammensetzungen enthalten 1 bis 30 Gew.-%, vorzugsweise 1,25 bis 27,5 Gew.-%, weiter bevorzugt 1,5 bis 25 Gew.-%, besonders bevorzugt 1,75 bis 22,5 Gew.-% und insbesondere 2 bis 20 Gew.-% $C_{12}$-$C_{18}$-Alkanole.

Es ist weiter bevorzugt, eine Mischung aus mindestens einem Fettalkohol mit 12 bis 18 Kohlenstoffatomen und mindestens einen Fettalkohol mit mehr als 18 Kohlenstoffatomen einzusetzen, wobei das Gewichtsverhältnis der Fettalkohole mit 12 bis 18 Kohlenstoffatomen zu den übrigen Fettalkoholen in einem Gewichtsverhältnisbereich von 1 zu 1 bis 2 zu 1 liegt.

Erfindungsgemäß bevorzugte Zusammensetzungen weisen eine Gesamtmenge an Fettalkohol jeweils bezogen auf das Gewicht der Gesamtzusammensetzung von kleiner als 12 Gew.-%, insbesondere kleiner als 10 Gew.-%, auf. In einer erfindungsgemäß besonders bevorzugten Zusammensetzung beträgt bezogen auf das Gesamtgewicht der Zusammensetzung die Gesamtmenge an Fettalkoholen 3 bis 12 Gew.-%, insbesondere 4 bis 10 Gew.-%.

Weitere bevorzugte erfindungsgemäße Zusammensetzungen sind dadurch gekennzeichnet, dass sie, beispielsweise zur Verbesserung der Konsistenz und der sensorischen Eigenschaften zusätzlich mindestens einen festen, wasserunlöslichen teilchenförmigen Füllstoff enthalten. In einer außerordentlich bevorzugten Ausführungsform ist dieser Füllstoff ausgewählt aus gegebenenfalls modifizierten Stärken (z. B. aus Mais, Reis, Kartoffeln) und Stärkederivaten, die gewünschtenfalls vorverkleistert sind (z.B. Dry FLO PC der Firma AKZO), Siliciumdioxid, Kieselsäuren, z. B. Aerosil®-Typen, sphärischen Polyalkylsesquisiloxan-Partikeln (insbesondere Aerosil® R972 und Aerosil® 200V von Degussa), Kieselgelen, Talkum, Kaolin, Magnesiumaluminiumsilikaten, Bornitrid, Lactoglobulinderivaten, z. B. Natrium-$C_{8-16}$-Isoalkylsuccinyllactoglobulinsulfonat, von Brooks Industries erhältlich als Handelsprodukt Biopol® OE, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat-oder (Meth)acrylat-Vinyliden-Copolymeren, die vernetzt sein können, oder Siliconen, sowie Mischungen dieser Substanzen.

Polymerpulver auf Basis eines Polymethacrylat-Copolymers sind z. B. als Handelsprodukt Polytrap® 6603 (Dow Corning) erhältlich. Andere Polymerpulver, z. B. auf Basis von Polyamiden, sind unter der Bezeichnung Orgasol® 1002 (Polyamid-6) und Orgasol® 2002 (Polyamid-12) von Elf Atochem erhältlich. Weitere Polymerpulver, die sich als erfindungsgemäß bevorzugte Füllstoffe eignen, sind z. B. Polymethacrylate (Micropearl® M von SEPPIC oder Plastic Powder A von NIKKOL), Styrol-Divinylbenzol-Copolymeren (Plastic Powder FP von NIKKOL), Polyethylen- und Polypropylen-Pulver (ACCUREL® EP 400 von AKZO) oder auch Siliconpolymere (Silicone Powder X2-1605 von Dow Corning).

Bevorzugte erfindungsgemäße Zusammensetzungen sind dadurch gekennzeichnet, dass sie mindestens einen festen, wasserunlöslichen teilchenförmigen Füllstoff in einer Gesamtmenge von 1 bis 99 Gew.-%, bevorzugt 2 - 90 Gew.-%, besonders bevorzugt 3 - 15 Gew.-%, außerordentlich bevorzugt 4, 5, 6, 7, 8, 9, 10, 11, 12, 13 und 14 Gew.-%, jeweils bezogen auf die Gesamtzusammensetzung, enthalten.

Weitere bevorzugte erfindungsgemäße Zusammensetzungen sind daher dadurch gekennzeichnet, dass sie zusätzlich mindestens ein lipophiles Verdickungsmittel enthalten.

Erfindungsgemäß bevorzugte lipophile Verdickungsmittel sind ausgewählt aus hydrophobierten Tonmineralien, pyrogenen Kieselsäuren, Ethylene/Propylene/Styrene Copolymeren, Butylene/ Ethylene/Styrene Copolymeren, Dextrinestern, Siliconelastomeren, unter Normalbedingungen festen Wachsen und/oder Glycerintriestern. Besonders bevorzugt enthält die erfindungsgemäße Zusammensetzung als lipophiles Verdickungsmittel zusätzlich mindestens ein Siliconelastomer. Bevorzugte hydrophobierte Tonmineralien sind ausgewählt aus hydrophobierten Montmorilloniten, hydrophobierten Hectoriten und hydrophobierten Bentoniten, besonders bevorzugt aus Disteardimonium Hectorite, Stearalkonium Hectorite, Quaternium-18 Hectorite und Quaternium-18 Bentonite. Die handelsüblichen Verdickungsmittel stellen diese hydrophobierten Tonmineralien in Form eines Gels in einer Ölkomponente, bevorzugt in Cyclomethicone und/oder einer Nichtsilicon-Ölkomponente, wie z. B. Propylencarbonat, bereit. Derartige Gele sind beispielsweise unter der Handelsbezeichnung Bentone® oder Thixogel erhältlich.

Erfindungsgemäß bevorzugte Zusammensetzungen sind dadurch gekennzeichnet, dass sie mindestens ein hydrophobiertes Tonmineral in einer Gesamtmenge von 0,5 - 10 Gew.-%, bevorzugt 1 - 7 Gew.-%, besonders bevorzugt 2 - 6 Gew.-%, außerordentlich bevorzugt 3 - 5 Gew.-%, jeweils bezogen auf das Gesamtgewicht der erfindungsgemäßen Zusammensetzung, enthalten.

Weitere erfindungsgemäß bevorzugte lipophile Verdickungsmittel sind ausgewählt aus pyrogenen Kieselsäuren, z. B. den Handelsprodukten der Aerosil®-Serie von Evonik Degussa. Besonders bevorzugt sind hydrophobierte pyrogene Kieselsäuren, besonders bevorzugt Silica Silylate und Silica Dimethyl Silylate.

Erfindungsgemäß bevorzugte Zusammensetzungen sind dadurch gekennzeichnet, dass sie mindestens eine pyrogene Kieselsäure, bevorzugt mindestens eine (bevorzugt hydrophobierte) pyrogene Kieselsäure, in einer Gesamtmenge von 0,5 - 10 Gew.-%, bevorzugt 1 - 7 Gew.-%, besonders bevorzugt 2-6 Gew.-%, außerordentlich bevorzugt 3 - 5 Gew.-%, jeweils bezogen auf das Gesamtgewicht der erfindungsgemäßen Zusammensetzung, enthalten.

Weitere erfindungsgemäß bevorzugte Zusammensetzungen sind dadurch gekennzeichnet, dass sie mindestens eine hydrophobierte pyrogene Kieselsäure und mindestens eine hydrophile Kieselsäure enthalten.

Weitere erfindungsgemäß bevorzugte lipophile Verdickungsmittel sind ausgewählt aus Ethylene/ Propylene/Styrene Copolymeren und Butylene/Ethylene/Styrene Copolymeren. Besonders bevorugt werden die Copolymere als vorverdicktes öl-basiertes Gel eingesetzt. Derartige Gele sind beispielsweise unter der Handelsbezeichnung Versagel® (ex Penreco) erhältlich. Bevorzugt sind Gele mit Mineralöl, hydriertem Polyisobuten, Isoparaffinen, wie Isohexadecan oder Isododecan, sowie mit Esterölen, insbesondere mit Isopropylpalmitat oder Isopropylmyristat.

Erfindungsgemäß bevorzugte Zusammensetzungen sind dadurch gekennzeichnet, dass sie mindestens ein Ethylene/Propylene/Styrene Copolymer und/oder Butylene/Ethylene/Styrene Copolymer in einer Gesamtmenge von 0,05 - 3 Gew.-%, bevorzugt 0,1 - 2 Gew.-%, besonders bevorzugt 0,2 - 1,0 Gew.-%, außerordentlich bevorzugt 0,3 - 0,5 Gew.-%, jeweils bezogen auf das Gesamtgewicht der erfindungsgemäßen Zusammensetzung, enthalten.

Weitere erfindungsgemäß bevorzugte lipophile Verdickungsmittel sind ausgewählt aus Siliconelastomeren. Eine weitere bevorzugte Ausführungsform der Erfindung ist dadurch gekennzeichnet, dass mindestens ein Siliconelastomer, das erhältlich ist durch die Vernetzung eines Organopolysiloxans, das mindestens 2 $C_2$-$C_{10}$-Alkenylgruppen mit terminaler Doppelbindung in jedem Molekül enthält, mit einem Organopolysiloxan, das mindestens 2 Silicon-gebundene Wasserstoffatome in jedem Molekül aufweist, enthalten ist.

Erfindungsgemäß besonders bevorzugte Organopolysiloxane mit mindestens 2 $C_2$-$C_{10}$-AlkenylGruppen mit terminaler Doppelbindung im Molekül sind ausgewählt aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen.

Erfindungsgemäß besonders bevorzugte vernetzende Organopolysiloxane mit mindestens zwei Silicon-gebundenen Wasserstoffatomen sind ausgewählt aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-siloxan-Copolymeren. Erfindungsgemäß besonders bevorzugte Siliconelastomere, die als Rohstoff bereits in einem bei Raumtemperatur unter Normalbedingungen flüssigen Silicon vorgequollen vorliegen und ein Silicon-basiertes Gel darstellen, sind kommerziell erhältlich, beispielsweise unter den Handelsnamen Dow Corning 9040 Silicone Elastomer Blend (ein Cyclomethicone (and) Dimethicone Crosspolymer von Dow Corning; Silikonelastomergehalt 12 - 13 Gew.-%), SFE 168, ein Cyclomethicone (and) Dimethicone/Vinyl Dimethicone Crosspolymer von GE Silicones, Vinyl Dimethicone Crosspolymere, enthalten in KSG-15 (Cyclomethicone (and) Dimethicone/Vinyl Dimethicone Crosspolymer, Siliconelastomergehalt 4-10 Gew.-%), KSG-16 (Dimethicone (and) Dimethicone/Vinyl Dimethicone Crosspolymer, Siliconelastomergehalt 20 - 30 Gew.-%), KSG-17 (Cyclomethicone (and) Dimethicone/Vinyl Dimethicone Crosspolymer), KSG-18 (Phenyl Trimethicone (and) Dimethicone/Phenyl Vinyl Dimethicone Crosspolymer, Siliconelastomergehalt 10 - 20 Gew.-%); and KSG-20, erhältlich von Shin Etsu Silicones of America (Akron, Ohio), und von Grant Industries Inc. (Elmwood Park, NJ) die Produkte aus der Gransil®-Serie, insbesondere Gransil SR-CYC (Cyclomethicone and Stearyl-Vinyl/hydromethylsiloxane Copolymer), Gransil® RPS Gel (INCI-Bezeichnung: Cyclopentasiloxane and Polysilicone-11), Gransil® GCM-4 (INCI-Bezeichnung: Cyclotetrasiloxane and Polysilicone-11), Gransil®GCM-5 (INCI-Bezeichnung: Cyclopentasiloxane and Polysilicone-11), Gransil® RPS (INCI-Bezeichnung: Cyclopentasiloxane and Polysilicone-11), GI-CD 10 (INCI-Bezeichnung: Cyclopentasiloxane (and) Stearoxymethicone/Dimethicone Copolymer (and) Dimethicone), Gransil® IDS (INCI-Bezeichnung: Isododecane (and) Cyclotetrasiloxane (and) Polysilicone-11), Gransil®PC-12

(INCI-Bezeichnung: Isododecane (and) Polysilicone-11), Gransil®IDS-5 (INCI-Bezeichnung: Isododecane (and) Cyclopentasiloxane (and) Polysilicone-11), Gransil®APK-1 (INCI-Bezeichnung: Dimethicone and Cyclopentasiloxane and Polysilicone-11 and Nylon-12 and Methyl Methacrylate/Acrylonitrile Copolymer and PEG-10 Dimethicone and Polysorbate-40 and Isohexadecane and Ammonium Polyacryloyldimethyl Taurate), Gransil®DMCM-5 (INCI-Bezeichnung: Dimethicone and Cyclopentasiloxane and Polysilicone-11), Gransil®DMG-6 mit Dimethicone (6 cSt) (INCI-Bezeichnung: Dimethicone and Polysilicone-11), Gransil®DMG-20 mit Dimethicone (20 cSt) (INCI-Bezeichnung: Dimethicone and Polysilicone-11), Gransil® AM-8 Gel (INCI-Bezeichnung: Caprylyl Methicone and Cyclopentasiloxane and Polysilicone-11), Gransil® DM 5 mit Dimethicone (5 cSt) (INCI-Bezeichnung: Dimethicone and Polysilicone-11), Gransil® DMID (INCI-Bezeichnung: Dimethicone and Isododecane and Polysilicone-11), Gransil® PM (INCI-Bezeichnung: Phenyl Trimethicone and Polysilicone-11), Gransil® ININ (INCI-Bezeichnung: Isononyl Isononanoate (and) Polysilicone-11).

Ebenfalls mit Vorzug in den erfindungsgemäßen Zusammensetzungen einsetzbar sind Siliconelastomere, die als Rohstoff bereits in einem bei Raumtemperatur unter Normalbedingungen flüssigen Silicon, gemischt mit einem Nicht-Silicon-haltigen Öl, Fett oder Wachs, vorgequollen vorliegen und ein Silicon-/Nicht-Silicon-basiertes Gel darstellen. Derartige Siliconelastomer-Zusammensetzungen sind ebenfalls kommerziell erhältlich, beispielsweise unter den Handelsnamen Gransil® MLB (INCI-Bezeichnung: Cyclopentasiloxane and Polysilicone-11 and Beeswax), Gransil® PS (INCI-Bezeichnung: Cyclotetrasiloxane and Polysilicone-11 and Petrolatum), Gransil® PS-5 (INCI-Bezeichnung: Cyclopentasiloxane and Polysilicone-11 and Petrolatum), Gransil® DMG-20 P mit Dimethicone (20 cSt) und Petrolatum (INCI-Bezeichnung: Dimethicone and Polysilicone-11 and Petrolatum), Gransil® RJO (INCI-Bezeichnung: Cyclopentasiloxane and Polysilicone-11 and Jojoba Oil), Gransil® LANO (INCI-Bezeichnung: Cyclopentasiloxane and Polysilicone-11 and Lanolin), Gransil® OHS-5 (INCI-Bezeichnung: Cyclopentasiloxane and Polysilicone-11 and Octyl Hydroxystearate) und Gransil® DML (INCI-Bezeichnung: Dimethicone (and) Neopentyl Glycol Diheptanoate (and) Polysilicone-11).

Eine weitere bevorzugte Ausführungsform der Erfindung ist dadurch gekennzeichnet, dass das Siliconelastomer durch die Vernetzung eines Organopolysiloxans, das mindestens 2 $C_2$- $C_{10}$-Alkenylgruppen mit terminaler Doppelbindung in jedem Molekül enthält, mit mindestens einem alpha, omega-Dien erhältlich ist. Erfindungsgemäß besonders bevorzugte vernetzende alpha, omega-Diene weisen die Formel $CH_2=CH(CH_2)_xCH=CH_2$ mit x = 1 - 20 auf. Besonders bevorzugte alpha, omega-Diene sind ausgewählt aus 1,4-Pentadien, 1,5-Hexadien, 1,6-Heptadien, 1,7-Octadien, 1,8-Nonadien, 1,11-Dodecadien, 1,13-Tetradecadien und 1,19- Eicosadien.

Erfindungsgemäß bevorzugte Zusammensetzungen sind dadurch gekennzeichnet, dass sie mindestens ein Siliconelastomer in einer Gesamtmenge von 0,05 - 3 Gew.-%, bevorzugt 0,1 - 2 Gew.-%, besonders bevorzugt 0,2 - 1,0 Gew.-%, außerordentlich bevorzugt 0,3 - 0,5 Gew.-%, jeweils bezogen auf das Gesamtgewicht der erfindungsgemäßen Zusammensetzung, enthalten.

Als ein bei Normalbedingungen festes Wachs enthält das erfindungsgemäße Mittel bevorzugt zusätzlich mindestens ein Polyethylenwachs. Bevorzugt geeignete Polyethylenwachse weisen 30 bis 60 Kohlenstoffatome auf.

Weitere bevorzugte erfindungsgemäße Zusammensetzungen sind dadurch gekennzeichnet, dass sie mindestens einen Duftstoff und/oder mindestens ein Parfümöl enthalten.

Als Duftstoffe oder Parfümöle können einzelne Riechstoffverbindungen, z.B. die synthetischen Produkte vom Typ der Ester, Ether, Aldehyde, Ketone, Alkohole und Kohlenwasserstoffe verwendet werden. Zu den phenolischen Riechstoffverbindungen zählt z. B. Carvacrol. Riechstoffverbindungen vom Typ der Ester sind z.B. Benzylacetat, Methylanthranilat, ortho-t-Butylcyclohexylacetat, p-tert.-Butylcyclohexylacetat, Diethylphthalat, Nonandiol-1,3-diacetat, iso-Nonylacetat, iso-Nonylformiat, Phenylethylphenylacetat, Phenoxyethylisobutyrat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat, Benzylsalicylat, Ethylsalicylat, iso-Amylsalicylat, Hexylsalicylat und 4-Nonanolid. Zu den Ethern zählen beispielsweise Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z.B. 6-Acetyl-1,1,3,4,4,6-hexamethyltetrahydronaphthalin, parat-Amylcyclohexanon, 2-n-Heptylcyclopentanon, β-Methylnaphthylketon und die lonone α-Isomethylionon und Methylcedrylketon, zu den Alkoholen Zimtalkohol, Anethol, Citronellol, Dimyrcetol, Eugenol, Geraniol, Linalool, Phenylethylalkohol und Terpineol, zu den Kohlenwasserstoffen gehören 1,3,4,6,7,8-Hexahydro-4,6,6,7,8,8-hexamethylcyclopenta-a-2-benzopyran, Hydroxymethylisopropylcyclopentan, 3-a-Methyldodecahydro-6,6,9a-trimethylnaphtho-2(2,1-b)furan, iso-Butylchinolin sowie die Terpene und Balsame. Bevorzugt werden Mischungen verschiedener Riechstoffe verwendet, die gemeinsam eine ansprechende Duftnote erzeugen.

Geeignete Parfümöle können auch natürliche Riechstoffgemische enthalten, wie sie aus pflanzlichen oder tierischen Quellen zugänglich sind, z.B. Pinien-, Citrus-, Jasmin-, Rosen-, Lilien- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Melissenöl, Minzenöl, Zimtblätteröl, Lindenblütenöl, Wacholderbeerenöl, Vetiveröl, Olibanöl, Galbanumöl, Laudanumöl, Gewürznelkenöl, iso-Eugenol, Thymianöl, Bergamotteöl, Geraniumöl und Rosenöl.

Bevorzugte erfindungsgemäße Zusammensetzungen enthalten mindestens einen Duftstoff und/ oder mindestens ein Parfümöl in einer Gesamtmenge von 0,01 - 10 Gew.-%, bevorzugt 0,1 - 5 Gew.-%, besonders bevorzugt 0,5 - 3 Gew.-%, außerordentlich bevorzugt 1 - 2 Gew.-%, jeweils bezogen auf das Gesamtgewicht der erfindungsgemäßen

Zusammensetzung.

Bevorzugte erfindungsgemäße Zusammensetzungen sind dadurch gekennzeichnet, dass sie zusätzlich mindestens einen Deodorant-Wirkstoff enthalten. Bevorzugte derartige Deodorant-Wirkstoffe sind ausgewählt aus Geruchsabsorbern, desodorierend wirkenden Ionenaustauschern, keimhemmenden Substanzen, präbiotisch wirksamen Substanzen sowie Enzyminhibitoren und, besonders bevorzugt, Kombinationen der genannten Deodorant-Wirkstoffe.

Silicate dienen als Geruchsabsorber, die auch gleichzeitig die rheologischen Eigenschaften der erfindungsgemäßen Zusammensetzung vorteilhaft unterstützen. Zu den erfindungsgemäß besonders vorteilhaften Silicaten zählen vor allem Schichtsilicate und unter diesen insbesondere Montmorillonit, Kaolinit, Ilit, Beidellit, Nontronit, Saponit, Hectorit, Bentonit, Smectit und Talkum. Weitere vorteilhafte Geruchsabsorber sind beispielsweise Zeolithe, Zinkricinoleat, Cyclodextrine, bestimmte Metalloxide, wie z. B. Aluminiumoxid, sowie Chlorophyll.

Unter keimhemmenden oder antimikrobiellen Wirkstoffen werden erfindungsgemäß solche Wirkstoffe verstanden, die die Zahl der an der Geruchsbildung beteiligten Hautkeime reduzieren bzw. deren Wachstum hemmen. Zu diesen Keimen zählen unter anderem verschiedene Spezies aus der Gruppe der Staphylokokken, der Gruppe der Corynebakterien, Anaerokokken und Mikrokokken.

Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Weiterhin sind Phenol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, $\alpha$-Monoalkylglycerinether mit einem verzweigten oder linearen gesättigten oder ungesättigten, gegebenenfalls hydroxylierten $C_6$-$C_{22}$-Alkylrest, besonders bevorzugt $\alpha$-(2-Ethylhexyl)glycerinether, im Handel erhältlich als Sensiva® SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar.

Weitere bevorzugte Deodorant-Wirkstoffe sind ausgewählt aus sogenannten präbiotisch wirksamen Komponenten, worunter erfindungsgemäß solche Komponenten zu verstehen sind, die nur oder zumindest überwiegend die geruchsbildenden Keime der Hautmikroflora hemmen, nicht aber die erwünschten, das heißt, die nicht-geruchsbildenden Keime, die zu einer gesunden Hautmikroflora gehören. Explizit sind hier die Wirkstoffe, die in den Offenlegungsschriften DE 10333245 und DE 10 2004 011 968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp.*, *Paullinia sp.*, *Panax sp.*, *Lamium album* oder *Ribes nigrum* sowie Mischungen dieser Substanzen.

Zu den Enzyminhibitoren gehören Stoffe, die die für die Schweißzersetzung verantwortlichen Enzyme, insbesondere die Arylsulfatase, $\beta$-Glucuronidase, Aminoacylase, Esterasen, Lipasen und/oder Lipoxigenase, hemmen, z. B. Trialkylcitronensäureester, insbesondere Triethylcitrat, oder Zinkglycinat. Bevorzugte erfindungsgemäße Zusammensetzungen sind dadurch gekennzeichnet, dass der mindestens eine zusätzliche Deodorant-Wirkstoff ausgewählt ist aus Arylsulfatase-Inhibitoren, $\beta$-Glucuronidase-Inhibitoren, Aminoacylase-Inhibitoren, Esterase-Inhibitoren, Lipase-Inhibitoren und Lipoxigenase-Inhibitoren, $\alpha$-Monoalkylglycerinethern mit einem verzweigten oder linearen gesättigten oder ungesättigten, gegebenenfalls hydroxylierten $C_6$ - $C_{22}$-Alkylrest, insbesondere $\alpha$-(2-Ethylhexyl)glycerinether, präbiotisch wirksamen Komponenten, Trialkylcitronensäureestern, insbesondere Triethylcitrat, Wirkstoffen, die die Zahl der an der Geruchsbildung beteiligten Hautkeime aus der Gruppe der Staphylokokken, Corynebakterien, Anaerokokken und Mikrokokken reduzieren bzw. deren Wachstum hemmen, Zinkverbindungen, insbesondere Zinkphenolsulfonat und Zinkricinoleat, Organohalogenverbindungen, insbesondere Triclosan, Chlorhexidin, Chlorhexidingluconat und Benzalkoniumhalogeniden, quartären Ammoniumverbindungen, insbesondere Cetylpyridiniumchlorid, Geruchsabsorbern, insbesondere Silikaten und Zeolithen, Natriumbicarbonat, Lantibiotika, sowie Mischungen der vorgenannten Substanzen.

Weitere bevorzugte erfindungsgemäße Zusammensetzungen sind dadurch gekennzeichnet, dass der mindestens eine zusätzliche Deodorant-Wirkstoff in einer Gesamtmenge von 0,1 - 10 Gew.-%, bevorzugt 0,2 - 7 Gew.-%, besonders bevorzugt 0,3 - 5 Gew.-%, bezogen auf das Gesamtgewicht der Aktivsubstanz in der Gesamtzusammensetzung, enthalten ist.

Antioxidative Stoffe können der oxidativen Zersetzung der Schweißkomponenten entgegenwirken und auf diese Weise die Geruchsentwicklung hemmen. Geeignete Antioxidantien sind Imidazol und Imidazolderivate (z. B. Urocaninsäure), Peptide wie z. B. D,L-Carnosin, D-Carnosin, L-Carnosin und deren Derivate (z. B. Anserin), Carotinoide, Carotine (z. B. $\alpha$-Carotin, $\beta$-Carotin, Lycopin) und deren Derivate, Liponsäure und deren Derivate (z. B. Dihydroliponsäure), Aurothioglucose, Propylthiouracil und weitere Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-,

Butyl-, Lauryl-, Palmitoyl-, Oleyl-, $\gamma$-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg bis $\mu$mol/kg), ferner Metallchelatoren (z. B. $\alpha$-Hydroxyfettsäuren, EDTA, EGTA, Lactoferrin), Huminsäuren, Gallensäure, Gallenextrakte, Catechine, Bilirubin, Biliverdin und deren Derivate, ungesättigte Fettsäuren und deren Derivate (z. B. $\gamma$-Linolensäure, Linolsäure, Arachidonsäure, Ölsäure), Folsäure und deren Derivate, Hydrochinon und dessen Derivate (z. B. Arbutin), Ubichinon und Ubichinol sowie deren Derivate, Vitamin C und dessen Derivate (z. B. Ascorbylpalmitat, -stearat, -dipalmitat, -acetat, Mg-Ascorbylphosphate, Natrium- und Magnesiumascorbat, Dinatriumascorbylphosphat und -sulfat, Kaliumascorbyltocopherylphosphat, Chitosanascorbat), Isoascorbinsäure und deren Derivate, Tocopherole und deren Derivate (z. B. Tocopherylacetat, -linoleat, -oleat und -succinat, Tocophereth-5, Tocophereth-10, Tocophereth-12, Tocophereth-18, Tocophereth-50, Tocophersolan), Vitamin A und Derivate (z. B. Vitamin-A-Palmitat), das Coniferylbenzoat des Benzoeharzes, Rutin, Rutinsäure und deren Derivate, Dinatriumrutinyldisulfat, Zimtsäure und deren Derivate, Kojisäure, Chitosanglycolat und -salicylat, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Selen und Selen-Derivate (z. B. Selenmethionin), Stilbene und Stilben-Derivate (z. B. Stilbenoxid, trans-Stilbenoxid). Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte, die diese Antioxidantien enthalten, eingesetzt werden.

Als lipophile, öllösliche Antioxidantien aus dieser Gruppe sind Tocopherol und dessen Derivate, insbesondere Tocopherylacetat, und Carotinoide sowie Butylhydroxytoluol/anisol bevorzugt.

Die Gesamtmenge der Antioxidantien in bevorzugten erfindungsgemäßen Zubereitungen beträgt 0,001 - 10 Gew.-%, vorzugsweise 0,05 - 5 Gew.-% und insbesondere 0,1 - 2 Gew.-%, bezogen auf die gesamte Zubereitung.

Auch komplexbildende Stoffe können die deodorierende Wirkung unterstützen, indem sie die oxidativ katalytisch wirkenden Schwermetallionen (z. B. Eisen oder Kupfer) stabil komplexieren. Geeignete Komplexbildner sind ausgewählt aus den vorstehend genannten Komplexbildnern.

Ein zweiter Gegenstand der Erfindung ist die Verwendung von Mischungen aus

(A) mindestens einem alkoxy-modifizierten Silikon, wie es bei der Beschreibung des ersten Erfindungsgegenstands definiert ist und
(B) mindestens einem Ester aus einer $C_8$-$C_{14}$-Alkansäure und einem $C_8$-$C_{14}$-Alkanol, wie er in einem der voranstehenden Ansprüche definiert ist, zur

Steigerung der schweißhemmenden Wirkung von Antitranspirantwirkstoffen (C) aus der Gruppe der Aluminium- oder Aluminium-Zirkonium-Salze.

Ein dritter Gegenstand der Erfindung ist die Verwendung Verwendung von Mischungen aus

(A) mindestens einem alkoxy-modifizierten Silikon, wie es bei der Beschreibung des ersten Erfindungsgegenstands definiert ist und
(B) mindestens einem Ester aus einer $C_8$-$C_{14}$-Alkansäure und einem $C_8$-$C_{14}$-Alkanol, wie er in einem der voranstehenden Ansprüche definiert ist, zur
Verbesserung der Textur von Antitranspirant-Stiften, welche mindestens einen Antitranspirantwirkstoff
(C) aus der Gruppe der Aluminium- oder Aluminium-Zirkonium-Salze enthalten.

Bezüglich bevorzugter Ausführungsformen der erfindungsgemäßen Verwendung gilt mutatis mutandis das zu den erfindungsgemäßen Mitteln (d.h. den Antitranspirant-Stiften) Gesagte.

Beispiele :

1. Versuchsreihe zur Textur und Schweißreduktion

Es wurden die folgenden Formulierungen hergestellt (alle Angaben in Gew.-% (Aktivsubstanz)) Antitranspirant Stifte

| | V1 | V2 | E |
|---|---|---|---|
| Aluminium-Zirkonium-Trichlorohydrex Gly (AAZG 531 Summit Reheis) (Gehalt Aktivsubstanz = 78,5 %) | 17,8 (13,97) | 17,8 (13,97) | 17,8 (13,97) |

(fortgesetzt)

| | V1 | V2 | E |
|---|---|---|---|
| Stearylalkohol | 19,5 | 19,5 | 19,5 |
| Cetylalkohol | 0,1 | 0,1 | 0,1 |
| PPG-14 Butyl Ether (Ucon Fluid AP, Dow) | 6,0 | 6,0 | 6,0 |
| Talkum | 3,0 | 3,0 | 3,0 |
| Hydrogeniertes Rizinusöl (Cutina HR, BASF) | 2,8 | 2,8 | 2,8 |
| Propylenglycol Dibenzoat (Lexfeel Shine, Inolex) | 2,0 | 2,0 | 2,0 |
| Myristyl Myristat (Tetradecyl tetradecanoat) | --- | 1,9 | 1,9 |
| PEG-12 Dimethicon (Xiameter OFX 0193 Fluid) | 4,0 | --- | 4,0 |
| Cyclopentasiloxan (Xiameter 0245) | ad 100 | ad 100 | ad 100 |
| Schweißreduktion (%) | - | 36 | 45 |
| Produktabgabe auf die Haut | zu gering | zu gering | gut |
| Hautgefühl | stumpf | cremig | cremig |

Die Rezepturen wurden in einem Sauna-Test auf ihre schweißhemmende Wirksamkeit überprüft. Dazu wurden die Produkte an 4 aufeinanderfolgenden Tagen in definierten Arealen auf dem Rücken von 25 Testpersonen angewendet. Ein dem jeweiligen Testfeld gegenüberliegendes Kontrollareal verblieb unbehandelt.

24 Stunden nach der letzten Produktanwendung wurde die Schweißbildung in einer Sauna bei 80°C für 15 Minuten ausgelöst. Zuvor wurden auf den Arealen saugfähige Pads angebracht, die am Ende der Schwitzphase zur Bestimmung der Schweißmenge gewogen wurden.

Die Tabelle zeigt, dass mit der erfindungsgemäßen Formulierung E im Vergleich zur Kontrolle V2 eine deutliche Verbesserung der Schweißreduktion erreicht werden konnte. Die Rezeptur V1 wurde hinsichtlich der Schweißreduktion nicht untersucht, die hier die Produktabgabe auf die Haut viel zu gering und das Hautgefühl zu stumpf waren.

Nur bei Kombination von Myristylmyristat mit PEG-12 Dimethicon und dem schweißhemmenden Wirkstoff (Formulierung E) wurden alle gewünschten Anwendungseigenschaften erzielt.

Der Stift mit der Formulierung E behielt auch nach wiederholter Anwendung eine cremige Textur mit optimaler Produktabgabe auf die Haut.

2. Formulierunqsbeispiele

Antitranspirant Stift

| Beispiel | 1 | 2 | 3 | 4 | 5 |
|---|---|---|---|---|---|
| Aluminium-Zirkonium-Trichlorohydrex Gly (AAZG 531, Summit Reheis) | 17,8 | 17,8 | 20,0 | --- | --- |
| Aluminium Chlorhydrat (Microdry 3115, Summit Reheis) | --- | --- | --- | 17,8 | 17,8 |
| Stearylalkohol | 19,5 | 19,5 | 19,5 | 19,5 | 19,5 |
| Cetylalkohol | 0,4 | 0,4 | 0,4 | 0,4 | 0,4 |
| Arachidylalkohol | 0,1 | 0,1 | 0,1 | 0,1 | 0,1 |
| PPG-14 Butyl Ether (Ucon Fluid AP, Dow) | 6,0 | 6,0 | 6,0 | 6,0 | 6,0 |
| Talkum | 3,0 | 3,0 | 3,0 | 3,0 | 3,0 |
| Hydrogeniertes Rizinusöl (Cutina HR, BASF) | 2,8 | 2,8 | 2,8 | 2,8 | 2,8 |
| Propylen Glycol Dibenzoat (Lexfeel Shine, Inolex) | 2,0 | 2,0 | 2,0 | 2,0 | 2,0 |
| Myristylmyristat (Crodamol MM, Croda) | 1,9 | 2,5 | 1,5 | 1,9 | 1,0 |
| PEG-12 Dimethicon (Xiameter OFX 0193 Fluid) | 4,0 | 3,0 | 6,0 | 4,0 | 1,0 |

(fortgesetzt)

| Beispiel | 1 | 2 | 3 | 4 | 5 |
|---|---|---|---|---|---|
| Cylopentasiloxan (Xiameter 0245) | ad 100 | ad 100 | ad 100 | ad 100 | ad 100 |

Antitranspirant Stift (Soft Solid)

| Beispiel | 6 | 7 | 8 | 9 |
|---|---|---|---|---|
| C20-C40 Alkohole (Performacol 425, Baker Petrolite) | 2,7 | 2,9 | 5,0 | 2,7 |
| Dow Corning 9040 Silicon Elastomer Blend | 0,6 | 5,0 | 3,0 | 0,6 |
| Stearylalkohol | 3,8 | 2,1 | 3,8 | 3,8 |
| Talkum | 2,0 | --- | --- | 2,0 |
| Aluminium Starch Octenylsuccinate (Dry Flo PC, National Starch) | 8,0 | --- | --- | 5,0 |
| Sorbitol / Sebacic Acid Copolymer Behenate (Syncrowax ORM-PW, Croda) | 1,8 | --- | --- | 3,0 |
| Silika (Aerosil 300, Evonik) | 0,5 | 0,5 | 0,5 | --- |
| Pentaerythrityl Distearate (Cutina PES, BASF) | 1,0 | 1,0 | 1,0 | --- |
| Aluminium Zirkconium Trichlorohydrex Gly (AAZG 531, Summit Reheis) | 20,0 | 20,0 | 20,0 | 20,0 |
| Myristyl Myristat (Crodamol MM, Croda) | 1,9 | 2,5 | 1,0 | 2,5 |
| PEG-12 Dimethicone (Xiameter OFX 0193 Fluid) | 4,0 | 6,0 | 1,0 | 3,0 |
| Cyclopentasiloxan (Xiameter 0245) | ad 100 | ad 100 | ad 100 | ad 100 |

## Patentansprüche

**1.** Antitranspirant-Stift, enthaltend

(A) mindestens ein alkoxy-modifiziertes Silikon,
(B) mindestens einen Ester aus einer $C_8$-$C_{14}$-Alkansäure und einem $C_8$-$C_{14}$-Alkanol und
(C) mindestens einen schweißhemmenden Wirkstoff aus der Gruppe der Aluminium- oder Aluminium-Zirkonium-Salze.

**2.** Stift nach Anspruch 1, **dadurch gekennzeichnet, dass** er ein alkoxy-modifiziertes Silikon (A) enthält, welches mindestens eine Struktureinheit der Formel (A1) besitzt,

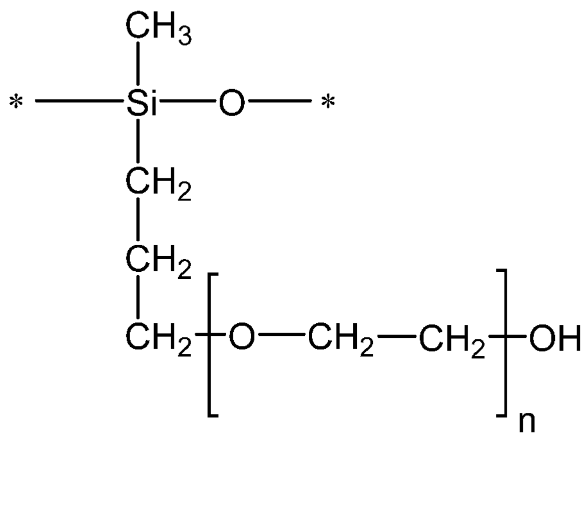

(A1)

worin n für eine ganze Zahl von 2 bis 20, bevorzugt für eine ganze Zahl von 4 bis 18, weiter bevorzugt für eine ganze Zahl von 6 bis 16, noch weiter bevorzugt für eine ganze Zahl von 8 bis 14 und ganz besonders bevorzugt für die Zahl 12 steht.

**3.** Stift nach einem der Ansprüche 1 bis 2, **dadurch gekennzeichnet, dass** er ein alkoxy-modifiziertes Silikon (A) enthält, welches sich aus Struktureinheiten der Formel (A1), der Formel (A2), der Formel (A3) und der Formel (A3') zusammensetzt,

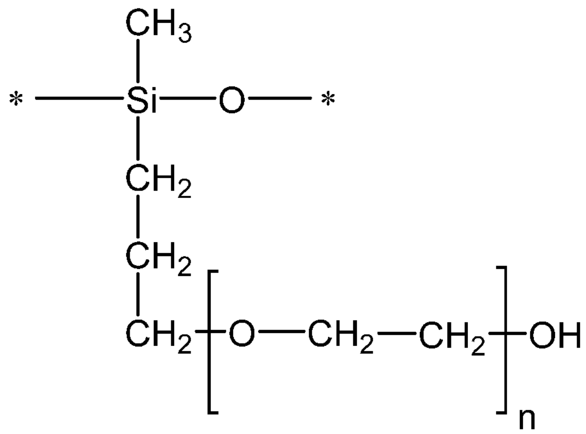
(A1)

worin n - unabhängig in jeder Struktureinheit (A1) - jeweils für eine ganze Zahl von 2 bis 20, bevorzugt für eine ganze Zahl von 4 bis 18, weiter bevorzugt für eine ganze Zahl von 6 bis 16, noch weiter bevorzugt für eine ganze Zahl von 8 bis 14 und ganz besonders bevorzugt für die Zahl 12 steht,

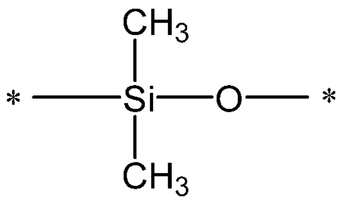
(A2)

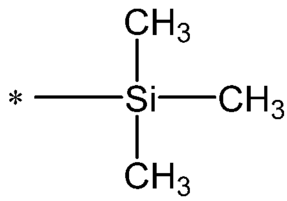
(A3).

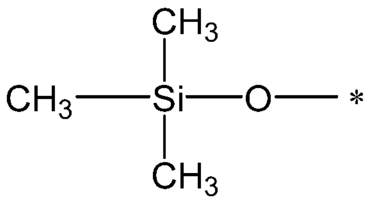
(A3‘).

**4.** Stift nach einem der Ansprüche 1 bis 3, **dadurch gekennzeichnet, dass** er mindestens ein alkoxy-modifiziertes Silikon (A) mit einer Molmasse von 800 bis 10 000 g/mol, bevorzugt von 1 000 bis 9 000 g/mol, weiter bevorzugt von 2 000 bis 8 000 g/mol und besonders bevorzugt von 2 500 bis 5 000 g/mol enthält.

**5.** Stift nach einem der Ansprüche 1 bis 4, **dadurch gekennzeichnet, dass** er einen Ester (B) der Formel (B1) enthält,

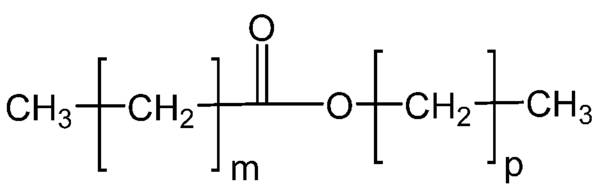
(B1)

worin

m für eine ganze Zahl von 6 bis 12, bevorzugt für eine ganze Zahl von 8 bis 12 und besonders bevorzugt für eine ganze Zahl von 10 bis 12 steht und
p für eine ganze Zahl von 7 bis 13, bevorzugt für eine ganze Zahl von 9 bis 13 und besonders bevorzugt für eine ganze Zahl von 11 bis 13 steht.

**6.** Stift nach Anspruch 5, **dadurch gekennzeichnet, dass** einen Ester (B) der Formel (B1) enthält, in welcher die Zahlen m und p die folgende Gleichung erfüllen: $m + 1 = p$

**7.** Stift nach einem der Ansprüche 1 bis 6, **dadurch gekennzeichnet, dass** er einen Wassergehalt von maximal 10,0 Gew.-%, bevorzugt von maximal 7,5 Gew.-%, weiter bevorzugt von maximal 5,0 Gew.-%, noch weiter bevorzugt von maximal 2,5 Gew.-% und besonders bevorzugt von maximal 1,0 Gew.-% - bezogen auf das Gesamtgewicht des Stiftes - besitzt.

**8.** Stift nach einem der Ansprüche 1 bis 7, **dadurch gekennzeichnet, dass** er ein oder mehrere alkoxy-modifizierte Silikone (A) in einer Gesamtmenge von 0,5 bis 10 Gew.-%, bevorzugt von 1,0 bis 8,0 Gew.-%, weiter bevorzugt von 1,8 bis 7,5 Gew.-% und besonders bevorzugt von 2,1 bis 5,2 Gew.-% - bezogen auf das Gesamtgewicht des Stiftes - enthält.

**9.** Stift nach einem der Ansprüche 1 bis 8, **dadurch gekennzeichnet, dass** er eine oder mehrere Ester (B) in einer Gesamtmenge von 0,5 bis 5,0 Gew.-%, bevorzugt von 1,0 bis 4,0 Gew.-%, weiter bevorzugt von 1,3 bis 3,5 Gew.-% und besonders bevorzugt von 1,6 bis 2,5 Gew.-% - bezogen auf das Gesamtgewicht des Stiftes - enthält.

**10.** Stift nach einem der Ansprüche 1 bis 9, **dadurch gekennzeichnet, dass** das Gewichtsverhältnis aus allen im Stift enthaltenen alkoxy-modifizierten Silikonen (A) zu allen im Stift enthaltenen Estern (B), d.h. das Gewichtsverhältnis (A)/(B), bei einem Wert von 1,0 bis 4,2, bevorzugt von 1,1 bis 4,0, weiter bevorzugt von 1,6 bis 3,2 und besonders bevorzugt von 1,8 bis 2,4 liegt.

**11.** Stift nach einem der Ansprüche 1 bis 10, **dadurch gekennzeichnet, dass** er einen oder mehrere schweißhemmende Wirkstoffe (C) in einer Gesamtmenge von 5,0 bis 20,0 Gew.-%, bevorzugt von 7,0 bis 17,5 Gew.-%, weiter bevorzugt von 8,5 bis 15,5 Gew.-% und besonders bevorzugt von 11,5 bis 14,5 Gew.-% - bezogen auf das Gesamtgewicht des Stiftes - enthält.

**12.** Stift nach einem der Ansprüche 1 bis 11, **dadurch gekennzeichnet, dass** das Gewichtsverhältnis aus allen im Stift enthaltenen schweißhemmenden Wirkstoffen (C) zur Summe der im Stift enthaltenen alkoxy-modifizierten Silikone (A) und Ester (B), d.h. das Gewichtsverhältnis (C)/[(A)+(B)], bei einem Wert von 1,0 bis 4,5, bevorzugt von 1,5 bis 3,5, weiter bevorzugt von 1,8 bis 3,1 und besonders bevorzugt von 1,9 bis 2,5 liegt.

**13.** Stift nach einem der Ansprüche 1 bis 12, **dadurch gekennzeichnet, dass** er zusätzlich ein oder mehrere Polyethylenglycole in einer Gesamtmenge von 0,01 bis 10,0 Gew.-%, bevorzugt von 0,07 bis 7,0 Gew.-%, weiter bevorzugt von 0,15 bis 4,5 Gew.-% und besonders bevorzugt von 0,25 bis 1,0 Gew.-% - bezogen auf das Gesamtgewicht des Stiftes - enthält.

**14.** Verwendung von Mischungen aus

(A) mindestens einem alkoxy-modifizierten Silikon, wie es in einem der voranstehenden Ansprüche definiert ist und
(B) mindestens einem Ester aus einer $C_8$-$C_{14}$-Alkansäure und einem $C_8$-$C_{14}$-Alkanol, wie er in einem der voranstehenden Ansprüche definiert ist, zur
Steigerung der schweißhemmenden Wirkung von Antitranspirantwirkstoffen (C) aus der Gruppe der Aluminium- oder Aluminium-Zirkonium-Salze.

**15.** Verwendung von Mischungen aus

(A) mindestens einem alkoxy-modifizierten Silikon, wie es in einem der voranstehenden Ansprüche definiert ist und
(B) mindestens einem Ester aus einer $C_8$-$C_{14}$-Alkansäure und einem $C_8$-$C_{14}$-Alkanol, wie er in einem der

voranstehenden Ansprüche definiert ist, zur Verbesserung der Textur von Antitranspirant-Stiften, welche mindestens einen Antitranspirantwirkstoff (C) aus der Gruppe der Aluminium- oder Aluminium-Zirkonium-Salze enthalten.

## Claims

1. An antiperspirant stick containing

   (A) at least one alkoxy-modified silicone,
   (B) at least one ester of a $C_8$-$C_{14}$ alkanoic acid and a $C_8$-$C_{14}$ alkanol, and
   (C) at least one antiperspirant active ingredient from the group of aluminum or aluminum-zirconium salts.

2. The stick according to claim 1, **characterized in that** it contains an alkoxy-modified silicone (A) which has at least one structural unit of formula (A1)

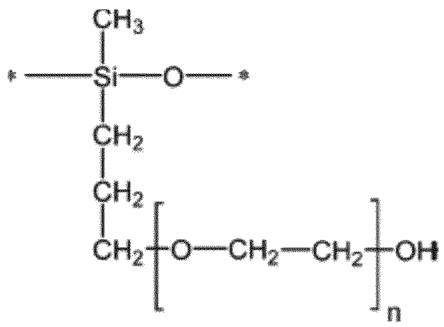

(A1),

where n represents an integer from 2 to 20, preferably an integer from 4 to 18, more preferably an integer from 6 to 16, even more preferably an integer from 8 to 14, and particularly preferably the number 12.

3. The stick according to one of claims 1 to 2, **characterized in that** it contains an alkoxy-modified silicone (A) which is composed of structural units of formula (A1), formula (A2), formula (A3) and formula (A3')

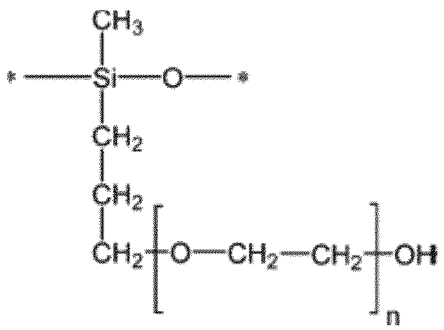

(A1),

where n represents in each case, independently in each structural unit (A1), an integer from 2 to 20, preferably an integer from 4 to 18, more preferably an integer from 6 to 16, even more preferably an integer from 8 to 14, and particularly preferably the number 12

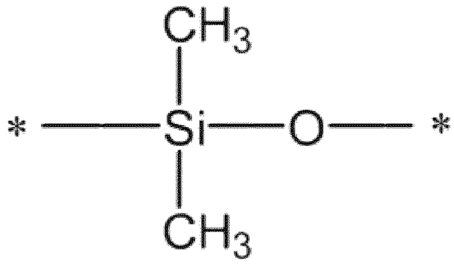
(A2)

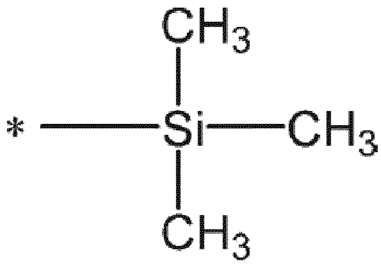
(A3).

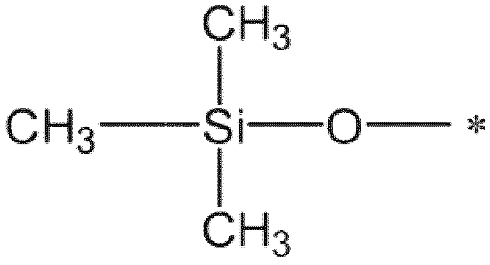
(A3').

4. The stick according to one of claims 1 to 3, **characterized in that** it contains at least one alkoxy-modified silicone (A) having a molar mass of from 800 to 10,000 g/mol, preferably from 1,000 to 9,000 g/mol, more preferably from 2,000 to 8,000 g/mol, and particularly preferably from 2,500 to 5,000 g/mol.

5. The stick according to one of claims 1 to 4, **characterized in that** it contains an ester (B) of formula (B1)

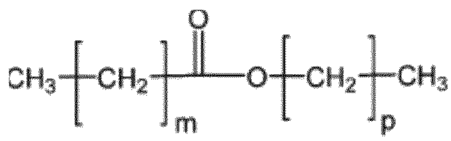
(B1),

where

m represents an integer from 6 to 12, preferably an integer from 8 to 12, and particularly preferably an integer from 10 to 12 and
p represents an integer from 7 to 13, preferably an integer from 9 to 13, and particularly preferably an integer from 11 to 13.

6. The stick according to claim 5, **characterized in that** it contains an ester (B) of formula (B1) in which the numbers m and p satisfy the following equation: m + 1 = p.

7. The stick according to one of claims 1 to 6, **characterized in that** it has a water content of at most 10.0 wt.%, preferably at most 7.5 wt.%, more preferably at most 5.0 wt.%, even more preferably at most 2.5 wt.%, and particularly preferably at most 1.0 wt.%, based on the total weight of the stick.

8. The stick according to one of claims 1 to 7, **characterized in that** it contains one or more alkoxy-modified silicones (A) in a total amount of from 0.5 to 10 wt.%, preferably from 1.0 to 8.0 wt.%, more preferably from 1.8 to 7.5 wt.%, and particularly preferably from 2.1 to 5.2 wt.%, based on the total weight of the stick.

9. The stick according to one of claims 1 to 8, **characterized in that** it contains one or more esters (B) in a total amount of from 0.5 to 5.0 wt.%, preferably from 1.0 to 4.0 wt.%, more preferably from 1.3 to 3.5 wt.%, and particularly preferably from 1.6 to 2.5 wt.%, based on the total weight of the stick.

10. The stick according to one of claims 1 to 9, **characterized in that** the weight ratio of all alkoxy-modified silicones (A) contained in the stick to all esters (B) contained in the stick, i.e. the weight ratio (A)/(B), has a value from 1.0 to 4.2, preferably from 1.1 to 4.0, more preferably from 1.6 to 3.2, and particularly preferably from 1.8 to 2.4.

**11.** The stick according to one of claims 1 to 10, **characterized in that** it contains one or more antiperspirant active ingredients (C) in a total amount of from 5.0 to 20.0 wt.%, preferably from 7.0 to 17.5 wt.%, more preferably from 8.5 to 15.5 wt.%, and particularly preferably from 11.5 to 14.5 wt.%, based on the total weight of the stick.

**12.** The stick according to one of claims 1 to 11, **characterized in that** the weight ratio of all antiperspirant active ingredients (C) contained in the stick to the sum of the alkoxy-modified silicones (A) and esters (B) contained in the stick, i.e. the weight ratio (C)/[(A)+(B)], has a value from 1.0 to 4.5, preferably from 1.5 to 3.5, more preferably from 1.8 to 3.1, and particularly preferably from 1.9 to 2.5.

**13.** The stick according to one of claims 1 to 12, **characterized in that** it additionally contains one or more polyethylene glycols in a total amount of from 0.01 to 10.0 wt.%, preferably from 0.07 to 7.0 wt.%, more preferably from 0.15 to 4.5 wt.%, and particularly preferably from 0.25 to 1.0 wt.%, based on the total weight of the stick.

**14.** The use of mixtures of

(A) at least one alkoxy-modified silicone, as defined in one of the preceding claims, and
(B) at least one ester of a $C_8$-$C_{14}$ alkanoic acid and a $C_8$-$C_{14}$ alkanol, as defined in one of the preceding claims,

for increasing the antiperspirant effect of antiperspirant active ingredients (C) from the group of aluminum or aluminum-zirconium salts.

**15.** The use of mixtures of

(A) at least one alkoxy-modified silicone, as defined in one of the preceding claims, and
(B) at least one ester of a $C_8$-$C_{14}$ alkanoic acid and a $C_8$-$C_{14}$ alkanol, as defined in one of the preceding claims,

for improving the texture of antiperspirant sticks which contain at least one antiperspirant active ingredient (C) from the group of aluminum or aluminum-zirconium salts.

**Revendications**

**1.** Stick antitranspirant contenant

(A) au moins un silicone modifié par un alcoxy,
(B) au moins un ester d'un acide $C_{8\text{-}14}$-carboxylique et d'un $C_{8\text{-}14}$-alcool, et
(C) au moins une substance active antitranspirante issue du groupe des sels d'aluminium ou d'aluminium-zirconium.

**2.** Stick selon la revendication 1, **caractérisé en ce qu'**il contient un silicone modifié par un alcoxy (A) possédant au moins une unité structurelle de formule (A1),

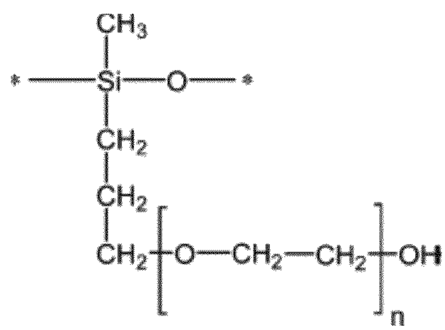

(A1)

où n est un nombre entier de 2 à 20, de préférence un nombre entier de 4 à 18, plus préférentiellement un nombre entier de 6 à 16, encore plus préférentiellement un nombre entier de 8 à 14 et tout particulièrement préférentiellement

12.

**3.** Stick selon une des revendications 1 à 2, **caractérisé en ce qu'**il contient un silicone modifié par un alcoxy (A) qui se compose d'unités structurelles de formule (A1), (A2), (A3) et (A3'),

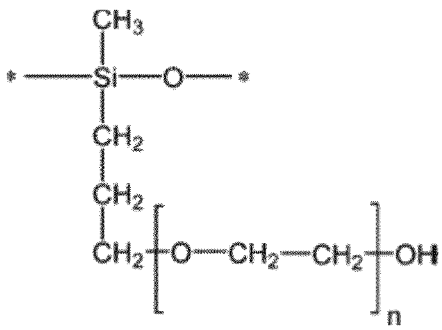

(A1)

où n - indépendamment dans chaque unité structurelle (A1) - est respectivement un nombre entier entre 2 et 20, de préférence un entier entre 4 et 18, plus préférentiellement un entier entre 6 et 16, encore plus préférentiellement un entier entre 8 et 14 et tout particulièrement préférentiellement 12,

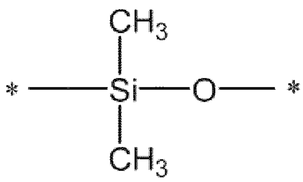

(A2)

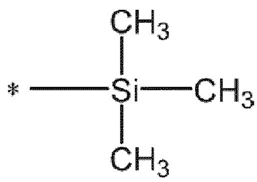

(A3).

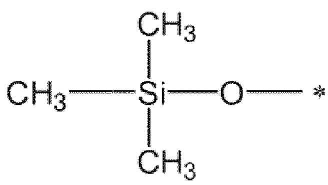

(A3').

**4.** Stick selon une des revendications 1 à 3, **caractérisé en ce qu'**il contient au moins un silicone modifié par un alcoxy (A) avec un poids molaire de 800 à 10 000 g/mol, de préférence de 1 000 à 9 000 g/mol, plus préférentiellement de 2 000 à 8 000 g/mol et particulièrement préférentiellement de 2 500 à 5 000 g/mol.

**5.** Stick selon une des revendications 1 à 4, **caractérisé en ce qu'**il contient un ester (B) de formule (B1),

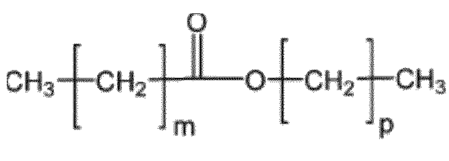

(B1;

où

m est un nombre entier entre 6 et 12, de préférence un nombre entier entre 8 et 12 et particulièrement préférentiellement un nombre entier entre 10 et 12, et
p est un entier de 7 à 13, de préférence un entier entre 9 et 13 et particulièrement préférentiellement un nombre entier entre 11 et 13.

6. Stick selon la revendication 5, **caractérisé en ce qu'**il contient un ester (B) de formule (B1) dans lequel les nombres m et p satisfont l'équation suivante : m+1=p

7. Stick selon une des revendications 1 à 6, **caractérisé en ce qu'**il a une teneur en eau de 10,0 % en poids maximum, de préférence 7,5 % maximum, plus préférentiellement de 5,0 % maximum, encore plus préférentiellement de 2,5 % maximum et particulièrement préférentiellement de 1,0 % maximum - rapporté au poids total du stick.

8. Stick selon une des revendications 1 à 7, **caractérisé en ce qu'**il contient au moins un silicone modifié par un alcoxy (A) à hauteur d'un total de 0,5 à 10 % en poids, de préférence de 1,0 à 8,0 % en poids, plus préférentiellement de 1,8 à 7,5 % en poids et particulièrement préférentiellement de 2,1 à 5,2 % en poids - rapporté au poids total du stick.

9. Stick selon une des revendications 1 à 8, **caractérisé en ce qu'**il contient au moins un ester (B) à hauteur d'un total de 0,5 à 5,0 % en poids, de préférence de 1,0 à 4,0 % en poids, plus préférentiellement de 1,3 à 3,5 % en poids et particulièrement préférentiellement de 1,6 à 2,5 % en poids - rapporté au poids total du stick.

10. Stick selon une des revendications 1 à 9, **caractérisé en ce que** le rapport pondéral de tous les silicones modifiés par un alcoxy (A) contenus dans le stick sur tous les esters contenus dans les esters (B), c'est-à-dire le rapport pondéral (A)/(B), est entre 1,0 et 4,2, de préférence entre 1,1 et 4,0, plus préférentiellement entre 1,6 et 3,2 et particulièrement préférentiellement entre 1,8 et 2,4.

11. Stick selon une des revendications 1 à 10, **caractérisé en ce qu'**il contient au moins une substance active antitranspirante (C) à hauteur d'un total de 5,0 à 20,0 % en poids, de préférence de 7,0 à 17,5 % en poids, plus préférentiellement de 8,5 à 15,5 % en poids et particulièrement préférentiellement de 11,5 à 14,5 % en poids - rapporté au poids total du stick.

12. Stick selon une des revendications 1 à 11, **caractérisé en ce que** le rapport pondéral de toutes les substances actives antitranspirantes (C) contenues dans le stick sur la somme des silicones modifiée par des alcoxy (A) et des esters (B) contenus dans le stick, c'est-à-dire le rapport pondéral (C)/[(A)+(B)], est entre 1,0 et 4,5, de préférence entre 1,5 et 3,5, plus préférentiellement entre 1,8 et 3,1 et particulièrement préférentiellement entre 1,9 et 2,5.

13. Stick selon une des revendications 1 à 12, **caractérisé en ce qu'**il contient, en plus, au moins un polyéthylène glycol à hauteur d'un total de 0,01 à 10,0 % en poids, de préférence de 0,07 à 7,0 % en poids, plus préférentiellement de 0,15 à 4,5 % en poids et particulièrement préférentiellement de 0,25 à 1,0 % en poids - rapporté au poids total du stick.

14. Utilisation de mélanges constitués de

(A) au moins un silicone modifié par un alcoxy comme défini dans une des revendications précédentes et
(B) au moins un ester d'un acide $C_{8-14}$-carboxylique et d'un $C_{8-14}$-alcool, comme défini dans une des revendications précédentes,

pour augmenter l'action antitranspirante de substances actives antitranspirantes (C) issues du groupe des sels d'aluminium ou d'aluminium-zirconium.

15. Utilisation de mélanges de

(A) au moins un silicone modifié par un alcoxy-modifié comme défini dans une des revendications précédentes et
(B) au moins un ester d'un acide $C_{8-14}$-carboxylique et d'un $C_{8-14}$-alcool, comme défini dans une des revendications précédentes

pour améliorer la texture de sticks antitranspirants contenant au moins une substance active antitranspirante (C) issue du groupe des sels d'aluminium ou d'aluminium-zirconium.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6074632 A
- DE 19756454 A1
- DE 10333245
- DE 102004011968